(19) **Europäisches Patentamt / European Patent Office / Office européen des brevets**

(11) **EP 3 240 897 B1**

(12) **FASCICULE DE BREVET EUROPEEN**

(45) Date de publication et mention de la délivrance du brevet:
**16.03.2022 Bulletin 2022/11**

(21) Numéro de dépôt: **15823699.2**

(22) Date de dépôt: **21.12.2015**

(51) Classification Internationale des Brevets (IPC):
**C12N 15/10** (2006.01)  **B32B 5/08** (2006.01)
**B32B 9/00** (2006.01)

(52) Classification Coopérative des Brevets (CPC):
**C12N 15/1013; B32B 5/08; B32B 9/00**

(86) Numéro de dépôt international:
**PCT/FR2015/053673**

(87) Numéro de publication internationale:
**WO 2016/108004 (07.07.2016 Gazette 2016/27)**

(54) **COMPLEXE MULTICOUCHES, PROCÉDÉ DE FABRICATION ET UTILISATION DU COMPLEXE**

MEHRSCHICHTIGER KOMPLEX, VERFAHREN ZUR HERSTELLUNG DIESES KOMPLEXES UND VERWENDUNG DIESES KOMPLEXES

MULTILAYER COMPLEX, METHOD FOR MANUFACTURING SAID COMPLEX AND USE OF SAID COMPLEX

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2014  FR 1463423**

(43) Date de publication de la demande:
**08.11.2017  Bulletin 2017/45**

(73) Titulaire: **Biomérieux**
**69280 Marcy-L'Etoile (FR)**

(72) Inventeurs:
- **BURR, Arnaud**
  **38950 Saint Martin le Vinoux (FR)**
- **LAAYOUN, Ali**
  **38260 La Frette (FR)**
- **LAURENT, Alain**
  **38100 Grenoble (FR)**
- **VEYRET, Raphäel**
  **38000 Grenoble (FR)**

(56) Documents cités:
| | |
|---|---|
| WO-A1-2014/090838 | US-A1- 2005 106 602 |
| US-A1- 2007 087 385 | US-A1- 2008 293 594 |
| US-A1- 2009 182 120 | US-A1- 2011 071 031 |

- **PRODELALOVA J ET AL: "Isolation of genomic DNA using magnetic cobalt ferrite and silica particles", JOURNAL OF CHROMATOGRAPHY, ELSEVIER SCIENCE PUBLISHERS B.V, NL, vol. 1056, no. 1-2, 12 novembre 2004 (2004-11-12), pages 43-48, XP004627928, ISSN: 0021-9673, DOI: 10.1016/J.CHROMA.2004.08.090**
- **NOORI ALI REZA ET AL: "Magnetic Nanoparticles Supported Ionic Liquids Improve Firefly Luciferase Properties", March 2014 (2014-03), APPLIED BIOCHEMISTRY AND BIOTECHNOLOGY, VOL. 172, NR. 6, PAGE(S) 3116-3127 ISSN: 0273-2289(print)**
- **SINGH G ET AL: "Magnetic silica beads functionalized with cobalt phthalocyanine for the oxidation of mercaptans in an alkali free aqueous medium", 2014, RSC ADVANCES 2014 ROYAL SOCIETY OF CHEMISTRY GBR, VOL. 4, NR. 55, PAGE(S) 29124 - 29130**
- **ZHANG L ET AL: "Fabrication and size-selective bioseparation of magnetic silica nanospheres with highly ordered periodic mesostructure", 23 October 2008 (2008-10-23), ADVANCED FUNCTIONAL MATERIALS 20081023 WILEY-VCH VERLAG DE, VOL. 18, NR. 20, PAGE(S) 3203 - 3212**

**EP 3 240 897 B1**

• EUNJI WOO ET AL: "Synthesis of magnetic/silica nanoparticles with a core of magnetic clusters and their application for the immobilization of His-tagged enzymes", JOURNAL OF MATERIALS CHEMISTRY ROYAL SOCIETY OF CHEMISTRY UK, vol. 20, no. 8, 2010, pages 1511-1515, ISSN: 0959-9428

**Description**

La présente divulgation concerne le domaine du diagnostic moléculaire. Plus précisément, elle concerne des complexes magnétiques solides permettant l'extraction et/ou la purification d'acides nucléiques (ADN et/ou ARN) à partir d'échantillons biologiques ou de prélèvements environnementaux, ces complexes étant au moins pourvus de trois couches distinctes.

L'essor de la biologie moléculaire a permis de faire d'immenses progrès dans le diagnostic. Ainsi, à partir d'un échantillon à tester, il est possible d'extraire et de détecter des acides nucléiques appartenant à l'hôte ou aux micro-organismes infectieux contenus dans l'échantillon. La détection, voire la quantification de ce matériel génétique permet d'établir un diagnostic par rapport à une infection microbienne ou à une présence d'oncogènes. Ceci se fait généralement en trois étapes décrites ci-dessous :

> 1) L'extraction d'acides nucléiques à partir d'échantillons biologiques complexes (sang, tumeur, aliments, etc..) qui consiste à une lyse chimique ou mécanique des cellules afin d'en libérer le contenu et particulièrement les acides nucléiques. Ces derniers vont être purifiés de manière sélective pour être ensuite amplifiés si leur quantité n'est pas suffisante pour une détection directe.
> 2) L'amplification des acides nucléiques purifiés par des techniques d'amplification de matériel génétique : NASBA, RT PCR, PCR... Cette étape est nécessaire quand la quantité d'acides nucléiques recueillie à partir d'un échantillon biologique est très faible ou que le test n'est pas suffisamment sensible pour une détection directe.
> 3) La détection des acides nucléiques amplifiés par des techniques dites en point final, en temps réel, par séquençage etc... Selon la technique de détection utilisée, cette étape peut permettre une quantification sélective des acides nucléiques cibles recherchés.

Pour une détection sensible et spécifique des acides nucléiques et donc réaliser un diagnostic le plus juste possible, il apparaît essentiel d'extraire et/ou isoler de façon efficace les acides nucléiques (ADN et ARN) des cellules. Cette étape d'extraction et/ou de purification, appelée aussi « Sample Prep » (pour sample préparation : préparation de l'échantillon) est généralement critique car de cette première étape va découler toute la qualité d'une série d'événements menant au résultat final du test de diagnostic. En effet, il est nécessaire d'avoir une extraction des acides nucléiques aussi spécifique et efficace (en quantité, en pureté et en temps) que possible afin de ne pas perdre de l'information, ce qui peut mener à un diagnostic erroné et être fatal pour un patient.

De nombreuses techniques ont été développées pour tenter d'extraire les acides nucléiques à partir de différents échantillons biologiques. Les méthodes les plus anciennes mettent en œuvre une multitude d'étapes généralement consistant à enrichir les cellules contenant les acides nucléiques, à lyser ces cellules, à séparer et éliminer les protéines, les membranes et autres constituants cellulaires, à purifier les acides nucléiques restants par précipitation dans des solvants organiques. Ces techniques sont coûteuses, demandent beaucoup de temps et elles sont souvent impossibles à automatiser. Elles ne sont donc plus adaptées aux pratiques actuelles où l'automatisation est nécessaire car elle permet d'obtenir les résultats le plus rapidement possible et évite les problèmes de contamination et d'erreurs humaines, particulièrement dans les cas de sepsis « infection du sang » où le pronostic vital d'un patient est engagé. Les techniques les plus récentes d'extraction d'acides nucléiques utilisent des phases solides où les cellules sont lysées dans des conditions de réactions spécifiques et les acides nucléiques libérés se lient à la phase solide. Il est bien connu dans l'état de l'art que les techniques actuelles d'extraction des acides nucléiques mettent très souvent en œuvre des phases solides qui sont des particules enrobées de silice. En effet, la silice a la propriété d'adsorber, de façon réversible, les acides nucléiques dans certaines conditions de concentration en sels et de pH, ce qui en fait un matériau très adapté à cet usage. Ces techniques sont clairement décrites dans « Rapid and simple method for purification of nucleic acids. », Boom, Journal of Clinical Microbiology, 1990 p495 et dans le brevet US 5 234 809 du même auteur.

Il est également connu d'utiliser des particules magnétiques enrobées de silice. La partie magnétique des particules sert le plus souvent à faciliter et à automatiser les étapes de capture, de lavage et d'élution des acides nucléiques car un simple aimant permet le déplacement des particules dans le tube et le prélèvement des surnageants pour des étapes de lavage. Les rendements d'extraction des acides nucléiques en sont nettement améliorés. Ces techniques sont bien décrites dans "Magnetic particles for the separation and purification of Nucleic acids", S. Berensmeier, Applied Microbial Biotechnology 2006 73 495-504; "The use of magnetic nanoparticles in the development of new molecular detection systems", I. J. Bruce, Journal of Nanosciences and nanotechnology et dans "Optimization of influencing factors of nucleic acid adsorption onto silica-coated magnetic particles: Application to viral nucleic acid extraction from serum", Ning Sun and al., Journal of Chromatography A, 2014, 1325, 31-39.

Bien que ces particules magnétiques de silice puissent être d'une grande efficacité dans l'extraction des acides nucléiques, leur protocole de fabrication est long, délicat et coûteux. En effet, les particules magnétiques enrobées de silice utilisées pour l'extraction des acides nucléiques peuvent parfois être très complexes à fabriquer, particulièrement au niveau de la couche de silice qui nécessite d'être parfaitement stable et contrôlée en épaisseur et en homogénéité.

La qualité et la nature de cette couche de silice a une importance fondamentale pour la qualité des acides nucléiques extraits ainsi que pour la reproductibilité des résultats d'une extraction des acides nucléiques à l'autre.

Ainsi, US 2010/0009375 A1 décrit la fabrication de particules magnétiques recouvertes d'une couche continue et ultrafine de silice inférieure à 1 nm pour l'extraction des acides nucléiques. Cette couche est cependant très difficilement mesurable et reste difficile à contrôler au niveau du procédé. Ceci rend le contrôle de la qualité de ces particules très complexe. Si la couche de silice est trop fine, les acides nucléiques peuvent s'adsorber sur la magnétite qui compose le cœur magnétique sans pouvoir être désorbés lors de l'étape d'élution.

US 2005/0287583 A1 et US 6924033 82 décrivent également la fabrication de particules magnétiques recouvertes d'une couche très épaisse de silice (ratio oxyde de silicium/oxyde de fer ($SiO_2/Fe_3O_4$) = 40/60) ce qui conduit à des agrégats dont la qualité et la reproductibilité d'un lot à l'autre peut sérieusement impacter la qualité de l'extraction. Ici également le procédé de fabrication est très complexe et implique un strict contrôle des ratios de réactifs $SiO_2$/magnétite/$Na_2O$.

Par ailleurs, US 2011/0186524 A1 décrit la fabrication de particules de silice magnétiques pour l'extraction des acides nucléiques, impliquant une étape de chauffage à 200°C pendant 7h et l'utilisation de solvants organiques, autant d'inconvénients en termes de temps de préparation, de coût et d'utilisation ultérieure (amplification, détection) des acides nucléiques extraits car, en fonction des traitements reçus lors de l'extraction, les rendements d'amplification et de détection peuvent être affectés. En outre, US2009/182120 décrit un complexe multi-couches constitué de particules magnétiques et de nanoparticules de silice pour l'extraction d'acides nucléiques.

Pour faire face à cette complexité de fabrication et éviter des procédés complexes pour maîtriser l'épaisseur de la couche d'enrobage de la magnétite, certains inventeurs ont proposé d'utiliser seulement la magnétite pour faire de l'extraction d'acides nucléiques à partir de milieux complexes. Ainsi, EP 1674571, US 6936414 et US 2007/0148651 décrivent l'extraction d'acides nucléiques par des particules constituées uniquement de la magnétite en milieu acide afin de favoriser l'interaction entre l'oxyde métallique et les acides nucléiques. Cependant, la désorption des acides nucléiques est très difficile et nécessite un chauffage des particules dans un tampon phosphate pour favoriser la compétition avec les acides nucléiques adsorbés et éluer les acides nucléiques adsorbés, ce qui impacte négativement le rendement d'extraction. Par ailleurs, l'utilisation d'agents dans le tampon d'élution qui favorise la désorption, comme le phosphate, peut entraîner une inhibition ultérieure de la PCR ou d'autres techniques d'amplification du matériel génétique extrait. Par conséquent, l'extraction des acides nucléiques avec des particules d'oxydes métalliques « nues » n'est pas satisfaisante.

Par ailleurs, certains inventeurs ont modifié les propriétés de particules d'oxydes métalliques en y greffant divers composés organiques ou inorganiques. Généralement, lesdites particules sont stabilisées par la complexation avec des dérivés de phosphates, phosphonates, carboxylates ou d'autres composés organiques. Ceci est décrit dans le livre « Stability constants of metal-ioncomplexes" de Lars Gunnar Sillén et Arthur Earl Martell édité en 1971 par la Chemical Society, dans US 5160725, par G. Pourroy dans Chem. Comm. 2010 46 985-987 ou dans Chem. Mater., 2008, 20 (18), pp 5869-5875. Ces complexes peuvent être utilisés en tant qu'agents de contraste en imagerie médicale, en tant que sonde pour détecter un brin d'ADN complémentaire *in vivo* ou en tant que ferrofluides bio-compatibles.

Seuls les travaux de Rittich Bohuslav dans « Isolation of genomic DNA using magnetic cobalt ferrite and silica particles », B. Rittch, J. of Chromatography A, 2004, 43-48 et de David Horack dans « Ferrite supports for the isolation of DNA from complex samples and polymerase chain reaction amplification », D. Horak, J. of Chromatography A, 2005, 93-98 traitent de particules magnétiques recouvertes de ligands pour l'extraction d'acides nucléiques. En revanche, les conditions d'utilisation ne sont pas adaptées à la lyse cellulaire et à la dénaturation des nucléases car ils n'utilisent pas de sels chaotropiques pour l'extraction. Ainsi, les rendements d'extraction et/ou d'amplification puis de détection des acides nucléiques ne sont pas optimaux. De plus, des problèmes d'élution des acides nucléiques sont observés.

Au vu de l'exposé de l'art antérieur ci-dessus, il apparaît clair qu'il était nécessaire de développer des supports solides magnétiques innovants permettant l'extraction et la purification des acides nucléiques et qui :

- soient faciles à synthétiser et peu coûteux, particulièrement au niveau de la couche recouvrant le composé magnétique,
- soient très performants en termes de rendement de capture et d'élution, d'extraction des acides nucléiques contenus dans des milieux biologiques complexes, en particulier le sang,
- permettent d'obtenir rapidement des acides nucléiques de grande pureté sans coélution d'inhibiteurs et qui soient à leur tour amplifiables avec efficacité,
- soient efficaces pour pouvoir capturer de très faibles quantités d'acides nucléiques ou de biomolécules,
- permettent d'ajuster la sélectivité de capture des acides nucléiques double-brin versus simple-brin,
- ne nécessitent pas l'utilisation de tampon d'élution pouvant inhiber. ultérieurement les réactions d'amplification ou d'analyse en aval de l'extraction.

Ainsi, l'objet de cette divulgation concerne le développement et la réalisation de nouveaux complexes solides permettant l'extraction et la purification d'acides nucléiques de façon efficace et ce à partir d'échantillons, de préférence d'échantillons biologiques complexes.

En premier lieu, la présente divulgation concerne un complexe tri-couches comprenant :

- une première couche comprenant au moins un composé magnétique,
- une deuxième couche recouvrant, au moins partiellement, la première couche et comprenant au moins un composé inorganique silicaté,
- une troisième couche recouvrant, au moins partiellement, la deuxième couche et comprenant au moins un composé ayant une affinité pour ledit au moins un composé magnétique et/ou pour ledit au moins un composé inorganique silicaté, ainsi que le procédé de fabrication d'un tel complexe tri-couches.

En second lieu, la présente invention concerne également l'utilisation de ce complexe tri-couches dans la purification de micro-organismes et/ou de biomolécules ou dans l'extraction de biomolécules, de préférence des acides nucléiques à partir d'un échantillon.

En troisième lieu, la présente invention concerne l'utilisation de ce complexe tri-couches dans la détection et/ou la quantification d'acides nucléiques cibles, à partir d'un échantillon susceptible de contenir lesdits acides nucléiques cibles, comprenant les étapes suivantes :

1. l'extraction des acides nucléiques d'un échantillon à l'aide de ce complexe tri-couches,
2. la détection et/ou la quantification des acides nucléiques cibles par des techniques de détection et/ou de quantification classiques.

En quatrième lieu, la présente invention concerne l'utilisation de ce complexe tri-couches dans une méthode de lyse de micro-organismes et/ou de cellules, à partir d'un échantillon, caractérisée en ce qu'elle consiste à mettre en contact au moins un échantillon avec au moins un complexe selon l'invention et dans lequel ledit au moins un composé inorganique silicaté et/ou ledit au moins un composé ayant une affinité pour ledit au moins un composé magnétique et/ou pour ledit au moins un composé inorganique silicaté comprend au moins un agent de la famille des détergents permettant la lyse.

En cinquième lieu, la présente invention concerne des trousses de diagnostic moléculaire comprenant au moins le complexe tri-couches selon l'invention.

La présence divulgation se propose donc de fournir des complexes tri-couches particulièrement utiles dans le domaine du diagnostic moléculaire. Les complexes selon la divulgation comprennent ou consistent en:

- une première couche comprenant au moins un composé magnétique,
- une deuxième couche recouvrant, au moins partiellement, la première couche et comprenant au moins un composé inorganique silicaté,
- une troisième couche recouvrant, au moins partiellement, la deuxième couche et comprenant au moins un composé ayant une affinité pour ledit au moins un composé magnétique et/ou pour ledit au moins un composé inorganique silicaté.

Par couche, on entend une épaisseur de matière ou d'une substance étendue de manière à former un film d'épaisseur variable. La couche peut être continue ou discontinue. La couche peut également être appelée revêtement.

Par complexe tri-couches, on entend un ensemble solide et compact constitué d'au moins trois couches distinctes physiquement et chimiquement. Dans le cadre d'un complexe multi-couches, les couches se superposent, la première couche est recouverte par la deuxième qui épouse la forme de la première couche, et la deuxième couche est elle-même recouverte par la troisième et ainsi de suite. La succession des trois couches se fait dans un ordre précis pour observer les propriétés du complexe. Lorsque la deuxième couche est discontinue, la troisième couche recouvre et est en contact direct à la fois avec la deuxième couche et la première couche. En revanche, la première couche ne peut pas recouvrir la deuxième ou la troisième couche, de même que la deuxième couche ne peut pas recouvrir la troisième couche.

Composé magnétique

Par composé magnétique, on entend un composé susceptible de réagir à un champ magnétique par une réaction d'orientation et/ou de déplacement dépendante de la force et de l'orientation dudit champ magnétique. Cette force s'effectue par l'intermédiaire du champ magnétique, et est produite par des charges en mouvement ou des aimants. Les composés magnétiques compris dans le complexe tri-couches selon l'invention sont choisis parmi des métaux et/ou des oxydes métalliques.

Le ou les métaux doués de capacités magnétiques utilisables dans le complexe tri-couches de la présente invention sont le fer, le cobalt, le nickel, les aciers, la fonte ainsi que les métaux réagissant plus faiblement au magnétisme comme le manganèse, le chrome, le platine et l'aluminium.

Le ou les oxydes métalliques utilisables dans la présente invention sont choisis parmi la magnétite, la maghémite et les ferrites de formule $MFe_2O_4$ avec M = Mn, Ni, Co, Zn ... ou tout autre métal classiquement utilisé dans les ferrites pouvant être dopés avec des atomes métalliques autres que le fer.

De préférence, le composé magnétique du complexe de l'invention est de la magnétite ou de la maghémite, de façon encore plus préférentielle de la magnétite.

Ledit au moins un composé magnétique du complexe de l'invention peut être sous forme plane et former une membrane, une plaque. Il peut également être sous forme variable, par exemple sous forme cubique, sous forme de cristal, d'aiguille, de cône, de sphère, sous forme d'une particule qui peut elle-même être sphérique, multiédrique, par exemple tétraédrique, octaédrique... La forme préférentielle du composé magnétique du complexe tri-couches de l'invention est la particule de dimension comprise entre 1 et 500nm, de préférence entre 2 et 400nm, plus préférentiellement entre 10 et 300 nm, plus préférentiellement encore entre 50 et 150nm.

Par particule, on entend une structure physique identifiable, distincte, isolée et non soluble dans un milieu aqueux ou un mélange de solvants organiques et aqueux ou dans un solvant organique et dont la taille peut être micrométrique ou nanométrique. De préférence, la particule est nanométrique et entre 1 et 400nm.

Lorsque ledit au moins un composé magnétique du complexe tri-couches est sous forme de particule alors l'ensemble du complexe tri-couches a la forme d'une particule car la couche de composé inorganique silicaté recouvrant ledit au moins un composé magnétique épouse la forme de ce dernier et il en est de même pour la troisième couche vis-à-vis de la deuxième couche. Dans le cas du complexe sous forme de particule, on peut dire que ledit au moins un composé magnétique forme le cœur de ladite particule puisqu'il est placé au centre de celle-ci.

Cependant, étant donné que les couches peuvent ne se recouvrir que partiellement, la forme finale du complexe tri-couches n'est pas nécessairement identique à la forme initiale dudit au moins un composé magnétique. Ainsi, avec un composé magnétique sous forme de particule, il est possible d'obtenir un complexe tri-couches sous une forme finale aléatoire. Dans une variante préférée de l'invention, le complexe tri-couches a la forme d'une mûre, le composé magnétique constituant le cœur solide et la deuxième couche formant une couche discontinue formée de nano-sphères satellites autour du cœur.

La couche de composé magnétique a une épaisseur micrométrique ou nanométrique.

Composé inorganique silicaté

Par composé inorganique silicaté, on entend tout composé comprenant de la silice et étant de nature inorganique. Le ou les composés inorganiques silicatés utilisable(s) dans le complexe tri-couches selon l'invention sont les silicates, les silicates de magnésium, de sodium, de potassium, de lithium ou de calcium, le talc, les alumino-silicates, le kaolin, la bentonite, les nanoparticules de silice, de préférence des nanoparticules de silice dont la taille est comprise entre 0,1 et 20 nm, de préférence entre 1 et 20 nm, les nanoparticules de silice mésoporeuses, les nanoparticules magnétiques recouvertes de silice, ainsi que les nanoparticules citées précédemment dont la silice est modifiée chimiquement avec des groupements organiques ou inorganiques.

Les groupements organiques ou inorganiques pouvant modifier la silice sont des composés ayant des groupements carbonés dotés de fonctions amines, carboxylique, thiol, alcool, acide phosphonique ou sulfonique, phosphonates et/ou phosphates, des composés de la famille des détergent comme les saponines, des homopolymères ou des copolymères, les polymères de l'anhydride maléique, le N-vinylpyrrolidone, les polyéthylène, les propylènes et les ethers de méthyl vinyl (AMVE) greffés avec de l'anhydride maléïque, le N-vinylpyrrolidone (NVP)/N-acryloxysuccinimide(NAS), les polysaccharides, les latex aminés.

Dans une variante préférée de réalisation du complexe tri-couches selon l'invention, le composé inorganique silicaté est de la bentonite ou des nanoparticules de silice dont la taille est comprise entre 0,1 et 20nm, encore plus préférentiellement entre 1 et 20 nm. Ces composés donnent les meilleurs résultats en terme d'extraction des acides nucléiques. Dans une variante de réalisation du complexe tri-couches selon l'invention, le composé inorganique silicaté est constitué par des nanoparticules de silice dont la silice est modifiée par liaison avec des saponines.

Ces composés donnent de bons résultats en terme de lyse cellulaire qui permettront en combinaison avec les complexes tri-couches préférés pour l'extraction d'acides nucléiques de permettre un diagnostic moléculaire efficace et précis.

La liaison entre la première couche du complexe de l'invention comprenant au moins un composé magnétique et la deuxième couche comprenant au moins un composé inorganique silicaté se fait par des liaisons électrostatiques ou covalentes, donnant lieu à une structure tridimensionnelle.

Dans une forme de réalisation particulièrement avantageuse du complexe tri-couches selon l'invention, ledit au moins un composé inorganique silicaté est sous forme particulaire tel qu'un grain, un feuillet, une aiguille, une fibre, une nanoparticule ... ou sous forme non-soluble dans un solvant aqueux, un mélange solvant aqueux-solvant organique ou un solvant organique.

Par exemple, la silice peut se trouver sous forme de grain ou de particule, la bentonite se trouve généralement sous forme de feuillet.

Les particules ou formes non solubles dans un solvant aqueux ou mélange solvant aqueux-solvant organique ou un solvant organique du composé inorganique silicaté ont généralement une taille nanométrique pour permettre une bonne accroche du au moins un composé inorganique silicaté à la couche d'au moins un composé magnétique. De préférence, la taille de ces particules ou formes non-solubles est comprise entre 0,1 et 20 nm, de préférence entre 1 et 20 nm, plus préférentiellement entre 2 et 10 nm, plus préférentiellement encore entre 6 et 8 nm.

La forme particulaire préférée du composé inorganique silicaté est la forme nano-particulaire. Ainsi, la première couche du complexe tri-couches est revêtue, au moins partiellement, d'une deuxième couche constituée de nano-particules de silice, de préférence de 0,1 à 20 nm, plus préférentiellement entre 1 et 20 nm, plus préférentiellement encore entre 2 et 10 nm et encore plus préférentiellement entre 6 et 8 nm. Dans un mode préféré de réalisation de l'invention, on utilise comme composé inorganique silicaté les nanoparticules de silice commercialisées par la société Sigma-Aldrich, appelées Ludox®, tout particulièrement, on préfèrera les nanoparticules Ludox®SM 7nm.

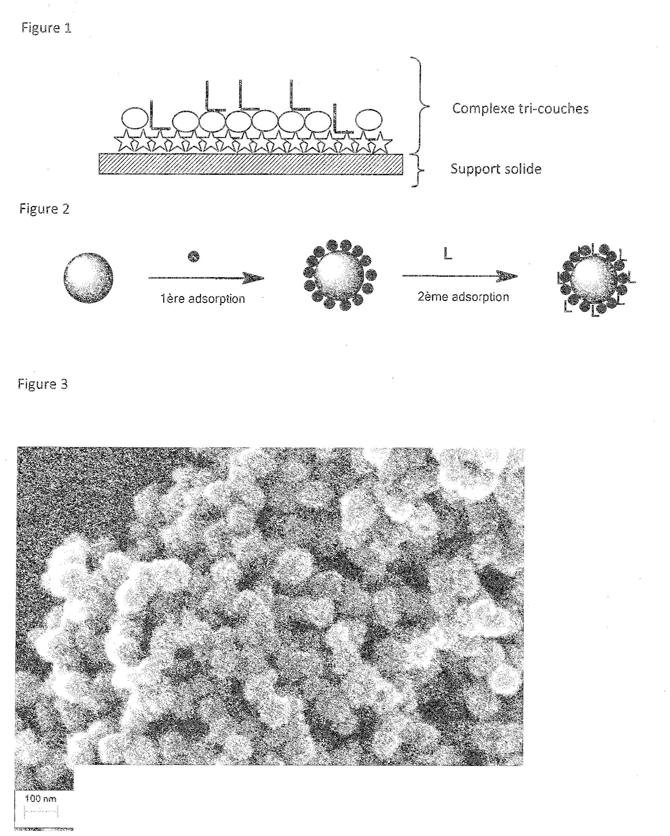

Par forme non-soluble dans un solvant aqueux ou mélange solvant aqueux-solvant organique ou dans un solvant organique, on entend une structure qui ne se dissout pas ou que partiellement dans ces solvants, quelle que soit la température du milieu. On peut aussi dire forme insoluble ou partiellement insoluble. Ainsi, même en conditions aqueuses, cette forme reste bien matérialisée, sous forme de suspension, structurée et identifiable. Elle peut être observée au microscope électronique. La figure 3 illustre des particules de magnétite (composé magnétique) recouvertes de nanoparticules de silice Ludox SM 7 nm (composé inorganique silicaté), elles-mêmes recouvertes de pyrophosphate (composé ayant une affinité pour ledit au moins un composé magnétique et/ou pour ledit au moins un composé inorganique silicaté). Cette figure exemplifie ainsi le complexe de l'invention avec le composé inorganique silicaté sous la forme non-soluble dans un solvant aqueux, dans un mélange solvant aqueux-solvant organique ou dans un solvant organique.

La couche de composé inorganique silicaté a une épaisseur nanométrique.

Composé ayant une affinité pour ledit au moins un composé magnétique et/ou pour ledit au moins un composé inorganique silicaté

Par composé ayant une affinité pour ledit au moins un composé magnétique et/ou pour ledit au moins un composé inorganique silicaté, on entend un composé capable de se lier au composé magnétique ou capable de se lier au composé inorganique silicaté, quelles que soient leurs formes respectives. Plus précisément, ce sont des molécules ayant une affinité pour les oxydes métalliques comme décrit dans « Stability constants of metal-ion complexes" de Lars Gunnar Sillén et Arthur Earl Martell édité en 1971 par la Chemical Society ». Ils peuvent être de nature organique ou inorganique. A titre d'exemple et façon non exhaustive, on peut citer l'acide citrique et ses sels, les ions phosphates, pyrophosphates, triphosphates, polyphosphates, les phosphonates et les acides phosphoniques, les phosphonates ou acides phosphoniques couplés à des molécules organiques, les composés de la famille des acides phosphoriques, des sulfonates, les composés de la famille des détergents et/ou les composés de la famille des acides carboxyliques.

Les molécules organiques couplées aux phosphonates ou acides phosphoniques peuvent être par exemple des riboses, des désoxyriboses, des acides aminés, des peptides,... Parmi les composés de la famille des détergents, on peut citer les saponines, les Tweens, les Tritons,...

On peut envisager d'avoir un complexe tri-couches selon l'invention dont la troisième couche est constituée par une combinaison de composés ayant une affinité pour ledit au moins un composé magnétique et/ou pour ledit au moins un composé inorganique silicaté cités ci-dessus, par exemple les ions phosphates, pyrophosphates ou triphosphates en combinaison avec une saponine. Ces molécules offrent alors plusieurs propriétés complémentaires. Elles peuvent ainsi en même temps lyser les cellules humaines de l'échantillon à tester et capturer/purifier des acides nucléiques ou des biomolécules de ces cellules humaines.

Il peut être envisagé d'utiliser différentes fonctionnalisations des deuxième et/ou troisième couches du complexe de l'invention pour effectuer une lyse sélective des cellules humaines et une capture sélective de bactéries, voire un enrichissement de micro-organismes cibles et/ou des acides nucléiques cibles ou biomolécules cibles. Lorsque la première couche est de la magnétite ou de la maghémite, ledit au moins composé ayant une affinité pour ledit au moins un composé magnétique et/ou pour ledit au moins un composé inorganique silicaté est un composé ayant une affinité pour le fer. Dans les variantes préférées de réalisation du complexe tri-couches selon l'invention, ledit au moins un composé ayant une affinité pour ledit au moins un composé magnétique et/ou pour ledit au moins un composé inorganique silicaté est constitué de monophosphates, de pyrophosphates, de triphosphates, d'acide citrique ou ses sels et/ou de saponine.

La liaison entre la deuxième couche du complexe de l'invention comprenant au moins un composé inorganique silicaté et la troisième couche comprenant au moins un composé ayant une affinité pour ledit au moins un composé magnétique et/ou pour ledit au moins un composé inorganique silicaté se fait par des liaisons électrostatiques ou covalentes ou de coordination donnant lieu à une structure tridimensionnelle.

Dans la forme de réalisation où la deuxième couche du complexe selon l'invention est constituée de particules ou de nanoparticules, de préférence de nanoparticules de silice, des interstices peuvent se produire entre lesdites particules ou nanoparticules et la troisième couche comprenant au moins un composé ayant une affinité pour au moins un composé magnétique adhère plus aisément et mieux à la première couche car certains atomes de métal et/ou d'oxydes métalliques de la première couche sont disponibles et plus accessibles pour former une réaction ou effectuer une liaison,

La couche de composé ayant une affinité pour le au moins un composé magnétique et/ou le au moins un composé inorganique silicaté a une épaisseur nanométrique ou inférieure.

Dans une forme de réalisation particulière, le complexe tri-couches selon l'invention et tel que défini ci-dessus comprend en outre un support solide sous toute ou partie de la première couche constituée par le au moins un composé magnétique.

Par support solide, on entend tout support capable de supporter la couche d'au moins un matériau magnétique. Il peut s'agir d'au moins un support plan, un support creux, une galette, une aiguille, une membrane, une plaque, une feuille, un cône, un tube, une bille, une particule... Le support est de préférence une bille ou une particule.

La liaison du support avec la première couche d'au moins un composé magnétique se fait par liaisons électrostatiques, par liaisons covalentes ou par tout autre moyen physique ou chimique. Par exemple, il est possible d'utiliser un adhésif pour fixer ledit au moins un composé magnétique au support ou une substance intermédiaire capable maintenir la liaison entre le support solide et le composé magnétique comme par exemple, une protéine, un polymère, un polymère hydrophile ou hydrophobe, une polydopamine, etc... Dans une forme de réalisation particulière, le complexe tri-couches tel que défini ci-dessus, avec ou sans support, est sous forme de particule et ladite troisième couche comprenant au moins un composé ayant une affinité pour ledit au moins un composé magnétique et/ou pour ledit au moins un composé inorganique silicaté se situe à l'extérieur de la particule. En quelques sortes, le cœur de la particule est constitué :

1) par le support qui est à son tour enrobé par le complexe tri-couches avec la première couche au contact du support, la deuxième couche en contact avec la première couche et la troisième couche en contact avec la deuxième couche ou la première couche, lorsque la deuxième couche est discontinue, et constituant la pellicule externe de la particule finale, ou

2) par ledit au moins composé magnétique enrobé par les deuxième et troisième couches, la troisième couche constituant l'enveloppe externe de la particule finale.

Dans cette variante de réalisation, la forme préférée est un complexe tri-couches ayant une forme de particule dont la première couche constitue le cœur de ladite particule et a une taille comprise entre 2 et 400 nm, de préférence entre 50 et 100 nm.

Les formes tout particulièrement préférées du complexe selon l'invention sont des complexes comprenant de la magnétite entre 50nm et 100nm (première couche), des nanoparticules de silice (Ludox SM 7nm tout préférentiellement) ou de la bentonite (deuxième couche) et des ions monophosphates, pyrophosphates, triphosphates (troisième couche).

Dans le complexe selon l'invention lorsqu'il est sous forme de particule, le ratio en poids par poids (ou w/w) entre ledit au moins un composé inorganique silicatéet ledit au moins un composé magnétique représente entre 0,1% et 60%, de préférence entre 1% et 50%, plus préférentiellement entre 3% et 35%, plus préférentiellement encore entre 4 et 10%. Dans les variantes préférentielles de réalisation de l'invention, le ratio en poids/poids de composé inorganique silicaté sur composé magnétique est plus élevé lorsque le composé inorganique silicaté est sous forme de feuillet, comme par exemple la bentonite, que lorsqu'il est sous forme de nanoparticules. Par exemple, lorsque la particule magnétite est recouverte de bentonite, le ratio bentonite/magnétite se situe entre 25% et 55% alors que si elle est recouverte de nanoparticules Ludox, le ratio Ludox/magnétite est entre 1% et 10%.

Dans le même contexte lorsque le complexe selon l'invention est sous forme de particule, le ratio molaire dudit au moins un composé magnétique (de préférence le Fer)/au moins un composé ayant une affinité pour ledit au moins un composé inorganique silicaté et/ou au moins un composé magnétique est compris entre 0,1% et 15%, de préférence entre 0,1 et 10%, plus préférentiellement entre 5% et 10% et plus préférentiellement encore entre 6% et 9%.

Méthode de production

Le complexe tri-couches selon l'invention est produit par des méthodes de fabrication faciles à mettre en œuvre et hautement reproductibles. Ceci permet d'avoir une fiabilité dans les performances du complexe produit ainsi qu'une uniformité, ou du moins très peu de variations, d'un lot de complexe produit à un autre.

De plus, les méthodes de production du complexe de l'invention mises en œuvre sont bon marché et certaines variantes de production sont très rapides.

Pour mettre en œuvre les méthodes de production du complexe selon l'invention, il convient tout d'abord de décrire les méthodes d'approvisionnement des différents constituants du complexe de l'invention.

*- Approvisionnement en composé <u>magnétique</u> porteur*

Ledit au moins un composé magnétique est soit trouvé à l'état naturel, soit il est synthétisé selon des protocoles classiques décrits dans la littérature soit par coprécipitation comme décrit par R. Massart (IEEE Trans. Magn. 1981, 17, p1247-1248.), soit par oxydation partielle de sels métalliques comme décrit par T. Sugimoto et E. Matjevic (Journal of Colloids and Interface Science, 1980, 74, P227-243), soit par décomposition de précurseurs organométalliques comme décrit par Maity (Journal of Magnetic Materials 321 1256 (2009)). L'avantage de ces techniques est que l'on peut facilement incorporer d'autres atomes métalliques comme le cobalt, le manganèse, le zinc, etc. de façon à obtenir des ferrites qui peuvent également être utilisés en tant que composé magnétique comme décrit ci-dessus dans le complexe de l'invention.

De façon alternative et préférée, ledit au moins un composé magnétique peut être acheté dans le commerce. C'est le cas notamment des nanoparticules d'oxydes métalliques qui peuvent être achetées chez des fournisseurs de pigments pour encre magnétiques comme décrit dans The Journal of Imaging Science and Technology November/December 2000, vol. 44, no. 6; p. 508-513 " The Influence of Particle Size, Shape and Particle Size Distribution on Properties of Magnetites for the Production of Toners*")*.

*- Approvisionnement en composé inorganique silicaté*

Ledit au moins un composé inorganique silicaté tel que décrit ci-dessus, par exemple du type magnésium ou sodium trisilicate, bentonite, kaolin, talc... peut être acheté facilement chez des fournisseurs de produits chimiques comme Sigma-Aldrich (Saint Louis, USA). Par ailleurs, de nombreux protocoles classiques et connus de l'homme du métier décrivent la façon d'obtenir ces composés inorganiques silicatés. Par exemple, les nanoparticules inorganiques silicatées, utilisées dans le mode de réalisation préféré de l'invention, peuvent être synthétisées selon des protocoles classiques de condensation du tetraéthoxy silane (TEOS) en milieu aqueux et alcalin qui sont décrits dans la littérature par W. Stöber (Journal of Colloids and Interface Science 1968, 26, p62-69). De façon alternative, les nanoparticules silicatées peuvent être obtenues chez des fournisseurs de produits chimiques comme la société Sigma-Aldrich (Saint Louis, USA.) fournissant par exemple les particules de la gamme LUDOX ou des équivalents de celles-ci ou comme la société NanoH (Lyon, France). Il est possible et facile d'introduire des groupements fonctionnels tels que amino, phosphonates, azido, alcyne etc... sur ces composés inorganiques silicatés (de préférence les nanoparticules) par co-condensation des silanes correspondants avec le TEOS de manière à accroître la force d'interaction avec le au moins un composé magnétique (par exemple des nanoparticules phosphonates).

*- Approvisionnement en composé ayant une affinité pour ledit au moins un composé magnétique et/ou pour ledit au moins un composé inorganique silicaté*

Ces composés ayant une affinité pour ledit au moins un composé magnétique et/ou pour ledit au moins un composé inorganique silicaté sont en général des produits facilement disponibles chez des fournisseurs de produits chimiques. Sinon, ils sont aisément préparés chimiquement par des méthodes classiques et connues de l'homme du métier.

La présente divulgation concerne une méthode de préparation d'au moins un complexe tri-couches tel que défini ci-dessus, caractérisée en ce qu'elle comprend au moins les étapes suivantes :

a0) éventuellement mettre en contact un support tel que défini ci-dessus avec au moins un composé magnétique tel que défini ci-dessus de sorte que le au moins un composé magnétique se fixe ou se lie au support,

a) mettre en contact le résultat de l'étape a0) ou au moins un composé magnétique tel que défini ci-dessus, avec au moins un composé inorganique silicaté tel que défini ci-dessus, de sorte qu'une interaction électrostatique et/ou une liaison covalente et/ou une liaison de coordination se fasse entre ledit au moins un composé magnétique et ledit au moins un composé inorganique silicaté et de sorte que la couche dudit au moins un composé inorganique silicaté recouvre, au moins partiellement, la couche dudit au moins un composé magnétique,

b) mettre en contact le complexe obtenu à l'étape a) (bi-couches constitué dudit au moins un composé magnétique/au moins un composé inorganique silicaté, avec ou sans support), avec au moins un composé ayant une affinité pour ledit au moins un composé magnétique et/ou pour ledit au moins un composé inorganique silicaté tel que défini ci-dessus, de préférence en milieu aqueux, de sorte que ledit au moins un composé ayant une affinité pour ledit au moins un composé magnétique et/ou pour ledit au moins un composé inorganique silicaté adhère et se positionne au-dessus de la couche dudit au moins un composé inorganique silicaté du complexe bi-couches, avec ou sans support, ou au-dessus du composé magnétique lorsque la couche du au moins un composé inorganique silicaté est discontinue.

Généralement, un ou plusieurs lavages dans de l'eau ou dans un milieu aqueux sont réalisés après chacune des étapes de production du complexe selon l'invention, de préférence après l'étape a) et b).

La liaison de la deuxième couche à la première couche se fait par adsorption et par liaison électrostatique et/ou par liaison covalente et/ou une liaison de coordination. Il en va de même pour la liaison de la troisième couche à la deuxième couche.

Dans une variante de réalisation, le complexe comprend un support sur lequel le complexe est fixé ou lié. Le support est tel que décrit ci-dessus. Dans ce cas, la méthode de fabrication du complexe sur le support peut se faire de plusieurs façons :

- soit le support est mis en contact avec le au moins un matériau magnétique de sorte qu'une interaction se fasse entre les deux matériaux, soit par liaisons électrostatiques, soit par liaison covalente, soit par liaison de coordination, puis la deuxième couche est ajoutée à la première couche fixée au support, la deuxième couche se fixant sur la première couche et non sur le support, puis la troisième couche est ajoutée au complexe bi-couches fixé au support,
- soit le support est mis en contact avec un complexe bi-couches constitué de la première couche liée à la deuxième couche, le support se fixant alors sur la première couche du complexe bi-couches de la même façon que ce qui est décrit ci-dessus, puis la troisième couche est ajoutée au complexe bi-couches fixé au support,
- soit le support est mis en contact avec le complexe tri-couches déjà constitué et il se fixe à la première couche du complexe tri-couches de la même façon que ce qui est décrit ci-dessus.

Le support a une épaisseur millimétrique à micrométrique.

On peut également utiliser un matériau permettant de fixer physiquement ou chimiquement la première couche du complexe au support. Il peut s'agir d'un adhésif ou de tout autre composé susceptible de remplir cette fonction, à savoir une substance intermédiaire capable de maintenir la liaison entre le support solide et le composé magnétique comme par exemple, une protéine, un polymère, un polymère hydrophile ou hydrophobe, une polydopamine, etc...

Dans une variante préférée de réalisation de la divulgation, le complexe final est sous forme de particule comprenant un cœur d'au moins un composé magnétique, lequel est recouvert au moins partiellement de deux couches : une couche d'au moins un composé inorganique silicatéet une couche de composé ayant une affinité pour ledit au moins un composé magnétique et/ou pour ledit au moins un composé inorganique silicaté, cette dernière couche se situant à l'extérieur du complexe.

Ainsi, la méthode de préparation du mode préféré de réalisation comprend au moins les étapes suivantes :

a) mettre en contact au moins un composé magnétique tel que défini ci-dessus avec au moins un composé inorganique silicaté tel que défini ci-dessus, ledit composé inorganique silicaté se trouvant déjà sous forme particulaire, nano-particulaire ou sous forme non-soluble, de sorte qu'une interaction électrostatique et/ou une liaison covalente et/ou une liaison de coordination se fasse entre lesdits au moins deux composés et que les particules ou formes non-solubles recouvrent au moins partiellement la particule d'au moins un composé magnétique constituant le cœur,
b) mettre en contact la particule enrobée obtenue à l'issue de l'étape a) avec au moins un composé ayant une affinité pour ledit au moins un composé magnétique et/ou pour ledit au moins un composé inorganique silicaté tel que défini ci-dessus, de préférence en milieu aqueux, de sorte qu'une interaction électrostatique et/ou une liaison covalente et/ou une liaison de coordination se fasse entre ledit au moins un composé ayant une affinité pour ledit au moins un composé inorganique silicaté et/ou ledit au moins un composé magnétique et ledit au moins un composé inorganique silicaté du complexe bi-couches obtenu à l'issue de l'étape a) ou ledit au moins un composé magnétique du complexe bi-couches obtenu à l'issue de l'étape a) et que ledit au moins un composé ayant une affinité pour ledit au moins un composé inorganique silicaté et/ou ledit au moins composé magnétique adhère à la surface dudit au moins un composé inorganique silicaté ou au cœur de la particule finale.

De préférence, l'interaction entre ledit au moins un composé magnétique et ledit au moins un composé inorganique silicaté est non covalente, de même pour l'interaction entre ledit au moins un composé ayant une affinité pour ledit au moins un composé inorganique silicaté et/ou ledit au moins un composé magnétique et ledit au moins un composé inorganique silicaté ou ledit au moins un composé magnétique.

Généralement, 'un ou plusieurs lavages dans de l'eau ou dans un milieu aqueux sont réalisés après chacune des étapes de production du complexe selon l'invention.

Le contact entre ledit au moins un composé magnétique et ledit au moins un composé inorganique silicaté se fait généralement à un pH compris entre 2 et 7, de préférence entre 3 et 6, tout préférentiellement entre 3 et 4. De façon générale, le revêtement de la première couche dudit au moins un matériau magnétique est favorisé par des interactions électrostatiques opposées. Ainsi, le revêtement doit être réalisé dans une zone de pH où le au moins composé Inorganique silicaté et ledit au moins un composé magnétique ont des charges de surface contraires comme décrit US 4280918.

Dans la forme préférée de réalisation du complexe de l'invention, la magnétite est utilisée comme composé magnétique et la silice est utilisée comme composé inorganique silicaté. Le point isoélectrique de la magnétite se situant à 6,8 et celui de la silice aux alentours de 2, le revêtement de la magnétite par la silice doit être effectué à un pH compris entre 2 et 6,8, de préférence à pH de 3,5.

Pour la méthode de fabrication du complexe, les températures utilisées se situent entre 15°C et 65°C, de préférence entre 20°C et 60°C utilisées.

Le mode de réalisation préféré de l'invention est très facile et rapide à synthétiser car il utilise des composés commerciaux ou facilement synthétisables.

Ainsi, dans un premier temps, ledit au moins un composé magnétique (de préférence les particules de magnétite) est mis à incuber entre pH 3 et 6 avec ledit au moins un composé inorganique silicaté (de préférence les nanoparticules inorganiques silicatées Ludox) afin de donner une charge positive audit au moins composé magnétique et une charge négative audit au moins composé inorganique silicaté. De cette manière, les deux types de composés (sous forme de particules dans la forme préférentiellement choisie de l'invention) se lient entre elles de façon très solide en formant une structure tridimensionnelle (en forme de mûre dans le cas de l'utilisation de la magnétite et des particules de Ludox).

Généralement, la température utilisée à cette première étape est la température ambiante mais la température peut varier entre 20°C et 60°C.

Les complexes composites obtenus, c'est-à-dire les complexes bi-couches (ou dans la forme préférentielle les particules recouvertes de Ludox) sont ensuite lavés par aimantation ou par toute techniques appropriée connue permettant d'obtenir la même fonction.

Dans un deuxième temps, on vient enrober au moins un composé ayant une affinité pour ledit au moins un composé magnétique et/ou pour ledit au moins un composé inorganique silicaté sur le complexe bi-couches constitué d'au moins un composé magnétique recouvert d'au moins un composé inorganique silicaté. Ceci se fait par incubation dudit complexe bi-couches avec ledit au moins un composé ayant une affinité pour ledit au moins un composé magnétique et/ou pour ledit au moins un composé inorganique silicaté en milieu aqueux pendant 30 secondes à 24h, de préférence 2h, à pH et température contrôlés.

Généralement, la température utilisée à cette étape est la température ambiante mais la température peut varier entre 20°C et -60°C.

Le complexe tri-couches est à nouveau lavé par aimantation ou toute autre technique connue ayant la même fonction, puis il peut être repris dans l'eau afin de déterminer la concentration par mesure de l'extrait sec.

Les lavages sont généralement effectués dans de l'eau ou au moins dans un milieu aqueux.

Cette méthode de revêtement de la couche d'au moins un composé <u>magnétique</u> avec au moins un composé inorganique silicaté et en particulier avec des formes non-solubles dans un solvant aqueux (particules inorganiques silicatées) est décrite dans US 4280918.

De façon originale, rapide et fiable, une des méthodes de fabrication du complexe particulièrement avantageuse est de mettre en œuvre au moins un composé magnétique, de préférence au moins une particule de magnétite ou de maghémite, et d'y déposer sur ce dernier ou de recouvrir au moins partiellement ce dernier d'au moins un composé inorganique silicaté, de préférence un composé inorganique silicaté de nature non soluble et déjà structuré comme des particules (par exemple des nanoparticules de silice) ou des feuillets (par exemple de la bentonite). Par exemple, comme composé inorganique silicaté, de préférence, on utilisera des nanoparticules de silices entre 0,1 et 20 nm, plus préférentiellement entre 1 nm et 20 nm et encore plus préférentiellement des particules Ludox® SM de 7nm. Lorsque l'on utilise ces types de composés, la fabrication du complexe est simplifiée car ces composés inorganiques silicatés sont disponibles dans le commerce. De plus, leur taille est parfaitement maîtrisée et donc la fabrication du complexe est très reproductible et standardisable. Dans cette variante de réalisation du complexe, une simple adsorption dudit au moins un composé inorganique silicaté sur ledit au moins un composé magnétique dans les bonnes conditions de pH suffit donc à générer un couche de silice de l'épaisseur désirée, maîtrisée et réglée par la taille des nanoparticules inorganiques silicatées choisies. Ensuite, lors du revêtement par la troisième couche, les manques entre les nanoparticules de matériau inorganique silicaté sont comblés par ledit au moins un composé ayant une affinité pour ledit au moins un composé magnétique et/ou pour ledit au moins un composé inorganique silicaté. Ceci est généralement le cas lorsque ledit au moins un composé formant la troisième couche a une affinité pour ledit au moins un composé magnétique préférentiellement. Lorsque le composé formant la troisième couche du complexe a une affinité pour ledit au moins un composé inorganique silicaté plus forte que pour le composé magnétique, il n'est pas indispensable qu'il y ait des manques dans la deuxième couche du complexe.

Il est à noter que de ce mode de réalisation du complexe implique une augmentation de la surface développée du complexe (ou surface spécifique) et donc un accroissement de la capacité d'extraction/purification des acides nucléiques.

De plus, en choisissant judicieusement des particules inorganiques silicatées dotées de fonctions remarquables (amines, acides carboxyliques, etc...), on peut facilement fonctionnaliser le composé magnétique avec ces fonctions sans avoir besoin de faire intervenir des processus complexes à maîtriser de condensation de silanes sur de la magnétite en présence de solvant(s) organique(s).

Afin d'offrir de très bonnes capacités de capture et d'extraction et/ou purification d'acides nucléiques, il est préférable que ledit au moins un composé magnétique soit entièrement revêtu par ledit au moins un composé inorganique silicaté.

Il peut être ainsi compris qu'un des avantages du complexe tri-couches selon l'invention réside dans la simplicité de mise en œuvre et de fabrication de celui-ci. Cependant, les avantages de ce complexe ne se limitent pas à sa fabrication, les avantages dans son utilisation sont multiples. En effet, de façon surprenante, il a été remarqué que le complexe selon l'invention offre des propriétés remarquables et particulièrement intéressantes dans la capture et l'élution d'acides nucléiques et en particulier dans des milieux ou échantillons biologiques complexes.

Les complexes tri-couches de l'invention ont été évalués pour leur capacité à extraire les acides nucléiques à partir de cellules ou de bactéries contenues dans un milieu ou échantillon complexe comme le sang.

Par échantillon, on entend un échantillon ayant diverses origines comme les échantillons d'origine alimentaire, environnementale, humaine, vétérinaire ou cosmétique.

Parmi les échantillons d'origine alimentaire, on peut citer, de façon non-exhaustive, un échantillon de produits lactés (yaourts, fromages, etc.), de viande, de poisson, d'œuf, de fruit, de légume, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales. Tous ces échantillons, s'ils ne sont pas liquides, sont préalablement traités pour être sous forme liquide.

Tel qu'indiqué précédemment, l'échantillon peut être d'origine environnementale et peut consister, par exemple, en un prélèvement de surface, d'eau, etc.

L'échantillon peut également consister en un échantillon biologique, d'origine humaine ou animale, pouvant correspondre à des prélèvements de fluide biologique (urine, sang total ou dérivés tels que sérum ou plasma, crachat ou salive, pus, liquide céphalorachidien, etc.), de selles (par exemple diarrhées cholériques), de prélèvements de nez, de gorge, de peau, de plaies, d'organes, de tissus ou de cellules isolées, d'échantillons d'écouvillonnage, des prélèvements ou lavages broncho-alvéolaires, des biopsies. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon analysé est, en général, susceptible de - ou suspecté de - contenir au moins une biomolécule représentative de la présence de microorganismes ou d'une maladie à détecter, caractériser ou suivre.

Par biomolécule, on entend un composé ou une entité chimique pouvant être un acide nucléique (ADN ou ARN de tout type ADN génomique, ADN complémentaire, ARN messager, ARN complémentaire, ARN de transfert, ARN mitochondrial, ADN de chloroplaste, ARN ribosomal, ADN plasmidique, ADN ou ARN viral, microARN, snoARN, siARN, ARNi, sous forme monocaténaire ou bicaténaire) ou une protéine.

Par un micro-organisme, on entend tout ou partie d'une bactérie, d'un champignon, d'une levure ou d'un virus.

Ainsi, la présente invention concerne également une méthode de purification de microorganismes et/ou biomolécules ou d'extraction de biomolécules, de préférence d'acides nucléiques, à partir d'un échantillon, de préférence biologique, dans laquelle on met en œuvre au moins un complexe tel que défini ci-dessus.

Par extraction, on entend une technique permettant d'isoler des biomolécules à partir d'un échantillon quel qu'il soit, par exemple, l'isolement d'ADN ou d'ARN à partir de cellules eucaryotes, procaryotes, animales humaines, de micro-organismes ou d'un tissu. Ainsi, l'extraction au sens de l'invention inclut la lyse et la purification des biomolécules. La purification comprend elle-même l'adsorption ou capture, le lavage et l'élution des biomolécules et/ou des micro-organismes. La capture consiste à adsorber les biomolécules et/ou micro-organismes sur le complexe et l'élution à la désorption ou libération de ces derniers du complexe selon l'invention. Lorsque l'on parle de la purification de micro-organismes, il n'y a pas de lyse de ces derniers, il peut s'agir alors d'un enrichissement en micro-organismes.

De préférence, les tests du complexe sont réalisés en conditions chaotropiques qui sont des conditions où les structures tridimensionnelles des macromolécules biologiques, comme les protéines, l'ADN ou l'ARN sont dénaturées. Les agents chaotropiques interfèrent avec les interactions intramoléculaires faibles (non-covalentes), comme les liaisons hydrogène, les forces de van der Waals et les forces hydrophobes. Parmi les agents chaotropiques, on peut citer l'urée, les sels de guanidine comme le chlorure ou le thiocyanate de guanidinium et le perchlorate de lithium. Ils sont généralement utilisés en concentration allant de 1 à 6 M, en particulier pour le GuSCN et le GuHCl.

Généralement on ajoute aussi un détergent qui aide à la lyse des cellules, ce dernier peut être choisi parmi les Tween, les tritons, le SDS et autres détergents communément utilisés à des concentrations comprises entre 0,05 et 5% en poids ou en volume par rapport au tampon de lyse,

Ainsi, les protéines capables de dénaturer ou endommager les acides nucléiques comme les nucléases sont inhibées ou détruites dans les conditions chaotropiques et ceci offre des conditions des plus favorables pour extraire de façon efficace des acides nucléiques. De préférence, pour la capture des acides nucléiques dans la présente invention, on utilise le chlorure de guanidinium ou le thiocyanate de guanidinium et/ou l'acide chlorhydrique (HCl) en milieu tamponné à pH 7 et un détergent, de préférence le triton X100.

Le complexe selon l'invention offre des propriétés hautement performantes dans la purification et/ou l'extraction des biomolécules et/ou micro-organismes et en particulier dans l'extraction des acides nucléiques.

Les bonnes propriétés de capture des acides nucléiques via des particules magnétiques enrobées de silice sont largement connues. Il est montré dans la présente demande que le fait d'ajouter un composé ayant une forte affinité pour ledit au moins un composé magnétique à une particule de composé magnétique affecte la capture des ADN. En revanche et de façon très surprenante, dans le complexe selon l'invention dans lequel nous avons un composé magnétique recouvert d'au moins un composé inorganique silicaté, lui-même recouvert par au moins un composé ayant une affinité pour ledit au moins un composé inorganique silicaté et/ou pour le au moins composé magnétique, soit une combinaison des deux types de particules déjà connues et décrites ci-dessus, la capture des acides nucléiques et en particulier des ADN n'est pas du tout affectée, elle en est même potentialisée. Cet effet synergique n'était absolument pas prévisible. Il était plutôt prévisible que ledit au moins un composé ayant une affinité pour ledit au moins un composé inorganique silicaté et/ou pour le au moins composé magnétique déplacent totalement le revêtement de composé inorganique silicaté et fasse chuter le rendement d'extraction de l'ADN. De même, il ne pouvait pas être envisagé des types de particules décrites précédemment que le complexe tri-couches améliorerait les capacités d'élution de l'ARN et offrirait ainsi des propriétés de purification et d'extraction des acides nucléiques ADN et ARN nettement améliorées par rapport à l'art antérieur. Les exemples illustrent très clairement ce phénomène et bien que les exemples soient limités à quelques composés magnétiques, composés inorganiques silicatés et composés ayant une affinité pour ledit au moins un composé inorganique silicaté et/ou pour ledit au moins composé magnétique, il est tout à fait possible pour l'homme du métier de généraliser les effets obtenus aux différentes combinaisons possibles et envisageables de ces trois composés listés ci-dessus pour former un complexe tri-couches selon l'invention.

Comme il a été précisé plus haut, la forme particulièrement préférée de composé inorganique silicaté est constituée par les nanoparticules de silice comprises entre 0,1 et 20 nm, plus préférentiellement entre 1 et 20 nm. Il faut noter que plus la taille des nanoparticules de composé inorganique silicaté liées à la première couche -couche magnétique- du complexe de l'invention est petite, plus l'extraction des acides nucléiques avec les complexes selon l'invention est importante et efficace. En quelques sortes, la taille de particules influe sur le rendement d'extraction des acides nucléiques et plus la surface spécifique des nanoparticules de composé inorganique silicaté est importante, plus l'extraction des acides nucléiques est bonne: un complexe ayant de la magnétite recouverte par des nanoparticules de composé inorganique silicaté Ludox® HS 40 captera moins d'acides nucléiques qu'un complexe ayant de la magnétite recouverte par des nanoparticules de composé inorganique silicaté Ludox® SM 7nm.

Pour obtenir de bons résultats dans la purification des micro-organismes et/ou des biomolécules ou l'extraction des biomolécules, en particulier des acides nucléiques, les ratios en poids par poids de composé inorganique silicaté / composé magnétique sont compris entre 0,1% et 60%, de préférence entre 0,5% et 30%, plus préférentiellement entre 1% et 20%, plus préférentiellement encore entre 2% et 15%, tout particulièrement préféré entre 3% et 10%.

Pour obtenir de bons résultats dans la purification des micro-organismes et/ou des biomolécules ou l'extraction des biomolécules, en particulier des acides nucléiques, les ratios en poids par poids de composé magnétique/ composé ayant une affinité pour ledit au moins un composé magnétique et/ou pour ledit au moins un composé inorganique silicaté sont compris entre 0,1% et 20%, de préférence entre 0,1% et 10%., plus . préférentiellement entre 4% et 8%.

Dans un autre de ses aspects, la présente invention concerne une méthode de détection et/ou de quantification des biomolécules, en particulier des acides nucléiques cibles, à partir d'un échantillon susceptible de contenir lesdits acides nucléiques cibles, comprenant les étapes suivantes :

1) l'extraction des acides nucléiques d'un échantillon par la mise en œuvre de la méthode telle que décrite ci-dessus,
2) la détection et/ou la quantification des acides nucléiques cibles par des techniques de détection et/ou de quantification classiques.

En effet, une fois que les biomolécules ont été extraites avec le complexe selon l'invention, il est intéressant de détecter les acides nucléiques cibles, voire de les quantifier afin de poser un diagnostic précis. Ces acides nucléiques cibles sont ceux suspectés ou susceptibles d'être présents dans l'échantillon testé.

Les méthodes de détection classiques utilisables sont toutes celles largement connues de l'homme du métier. Par exemple, on peut citer de façon non exhaustive des marquages radio-actifs; des marquages froids: colorimétrie, fluorescence, chimioluminescence; des hybridations moléculaires ; des Southern Blot; des Northern Blot; des Dot Blot ou de l'hybridation in situ. De façon préférentielle, on utilise une détection avec une sonde détection.

Par sonde de détection ou sonde, on entend une séquence nucléique d'un enchaînement nucléotidique de quatre bases de types différents choisis parmi le groupe d'adénine, thymine, guanine, uracile, cytosine, qui est capable de s'hybrider spécifiquement sur un amplicon et comportant au moins un marqueur. La sonde peut être une sonde de forme arrondie (appelée O-probe, voir demande de brevet de la Demanderesse FR08/54549 déposée le 4 juillet 2008), une balise moléculaire (autrement appelée Molecular Beacon), une sonde Taqman® ou une sonde FRET. Ces trois derniers types de sondes sont bien connus de l'homme du métier. Ces sondes peuvent éventuellement être constituées en tout ou partie de nucléotides modifiés. Chaque sonde comporte un marqueur et éventuellement un quencher. Parmi ces sondes, préférentiellement, on utilise des sondes émettant une fluorescence lorsqu'elles s'hybrident à la séquence complémentaire, des sondes décrites par Tyagi & Kramer (Nature Biotech, 1996, 14 :303-308), communément connues sous le nom de « molecular beacons » ou des kits commerciaux comme par exemple des kits de la gamme R-gene® de chez Argene (bioMérieux, Verniolle, France). Il est possible de faire la détection en temps réel ou à la fin de la réaction.

Les méthodes de quantification utilisées sont les méthodes de quantification standards et classiquement utilisées par l'homme du métier. Par exemple, il est possible d'utiliser des kits de quantification de gamme R-gene® de chez Argene (bioMérieux, Verniolle, France). Ces méthodes font classiquement intervenir des gammes de quantification standards (QS) permettant d'évaluer la quantité réelle d'un acide nucléique donné ou d'acides nucléiques dans l'échantillon testé.

Dans une variante de réalisation, la présente invention concerne une méthode de détection et/ou de quantification d'acides nucléiques cibles telle que décrite ci-dessus dans laquelle entre l'étape d'extraction et l'étape de détection des acides nucléiques, il y a une étape d'élution des acides nucléiques du complexe mis en œuvre dans l'étape d'extraction et/ou une étape d'amplification des acides nucléiques par des techniques classiques.

L'étape d'élution se fait généralement à l'aide d'une solution à pH légèrement alcalin et de faible force ionique à une température comprise entre 50°C et 70°C. De façon générale, on préfère utiliser les produits commerciaux tels que les produits de la gamme d'extraction NucliSENS easyMAG (bioMerieux, France). Ces conditions sont des conditions classiques permettant de conserver l'intégrité des acides nucléiques et sont des conditions standards.

Par amplification ou réaction d'amplification, on entend toute technique d'amplification d'acides nucléiques bien connue par l'homme du métier

L'étape d'amplification se fait généralement car la quantité des acides nucléiques à détecter et/ou quantifier est très faible et qu'il est nécessaire de passer par une phase d'amplification de ces derniers pour les détecter et/ou les quantifier afin de donner un diagnostic précis. Sans phase d'amplification, il est tout à fait possible d'obtenir des résultats erronés concluant à l'absence d'un acide nucléique cible dans un échantillon alors qu'en réalité il est présent dans ce dernier mais en quantité tellement faible que les techniques utilisées ne permettent pas de le détecter. Ainsi de nombreuses méthodes d'amplification peuvent être utilisées telles que PCR, RT-PCR, LCR, RCR, 3SR, RCA, NASBA, TMA, SDA ou toutes techniques d'amplification des acides nucléiques connues de l'homme du métier.

Les tests réalisés de détection et/ou amplification d'acides nucléiques cibles avec les complexes selon l'invention donnent d'excellents résultats.

Dans un autre de ses aspects, la présente invention concerne une méthode de lyse de micro-organismes et/ou de cellules et/ou de tissus, à partir d'un échantillon, caractérisée en ce qu'elle consiste à mettre en contact au moins un échantillon avec au moins un complexe tri-couches tel que décrit ci-dessus et dans lequel ledit au moins un composé inorganique silicaté et/ou ledit au moins un composé ayant une affinité pour ledit au moins un composé magnétique et/ou pour ledit au moins un composé inorganique silicaté comprend au moins un agent de la famille des détergents permettant la lyse.

Dans certaines situations d'analyse, il peut être intéressant de lyser de façon ciblée des cellules, les cellules d'un tissu et/ou des micro-organismes. Ainsi, l'utilisation de ce complexe peut être intéressante lorsqu'il s'agit de cibler un certain type cellulaire ou de micro-organisme. Par exemple, dans le cadre du sepsis, il peut être nécessaire de lyser, de façon ciblée et sélective, les membranes et les acides nucléiques des cellules sanguine humaines présentes en grande quantité dans un échantillon. Ceci peut être réalisé avec de la saponine qui pénètre et fragilise les membranes et les enveloppes contenant du cholestérol. Il peut être nécessaire de faire cette lyse sélective en vue de pouvoir sélectionner et extraire les micro-organismes pathogènes présents en très faibles quantités dans l'échantillon testé. Après une lyse de ces micro-organismes très faiblement représentés, il sera possible de purifier leurs acides nucléiques et de les amplifier de façon sélective avec une très haute sensibilité malgré le fait qu'ils soient présents en très petite quantité dans l'échantillon initial. Le complexe tri-couches selon l'invention pourrait aider en ce sens. Comme il a été décrit ci-dessus, les composés constituant la deuxième et/ou la troisième couche du complexe de l'invention peuvent être fonctionnalisés avec des composés de la famille des détergents, comme les saponines par exemple. Ainsi, ces complexes ainsi fonctionnalisés peuvent avoir un rôle dans la lyse sélective de cellules humaines. Ainsi, le complexe tri-couches pourrait être utilisé pour faire une première étape de lyse et capture sélective des acides nucléiques non ciblés et faciliter en quelques sorte une étape d'enrichissement ciblé de microorganismes présents dans l'échantillon mais non captés par les complexes de l'invention. Il peut également être envisagé d'avoir un complexe tri-couches selon l'invention dont ledit au moins un composé inorganique silicaté et/ou ledit au moins un composé ayant une affinité pour ledit au moins un composé magnétique et/ou pour ledit au moins un composé inorganique silicaté soient fonctionnalisés de façons différentes et complémentaires et permettent de capter et/ou lyser différents types de biomolécules et/ou micro-organismes.

Dans le mode de réalisation préféré selon cette méthode, ledit au moins un composé inorganique silicaté comprend des nanoparticules de silice entre 0,1 nm et 20 nm, plus préférentiellement entre 1 nm et 20 nm, dont la silice est liée avec des saponines et/ou le composé ayant une affinité pour ledit au moins un composé magnétique et/ou pour ledit au moins un composé inorganique silicaté est couplé à au moins une saponine. La fonctionnalisation peut ne pas être complète, c'est-à-dire que les composés ainsi fonctionnalisés peuvent ne recouvrir que partiellement ledit au moins un composé magnétique.

Tous les composés inorganiques silicatés et tous les composés ayant une affinité pour le au moins composé magnétique et/ou pour le composé inorganique silicatélistés ci-dessus sont « fonctionnalisables » par un détergent de type saponine. Dans une forme de réalisation préférée pour l'utilisation de lyse cellulaire ou de micro-organismes sélective, le composé inorganique silicaté est une nanoparticules de silice Ludox® 7nm et le composé ayant une affinité pour le au moins un composé magnétique et/ou le au moins un composé inorganique silicaté est choisi parmi la dopamine et ses dérivés, les catechols et leurs dérivés, les acides phosphoniques, les phosphonates, les phosphates et les composés de la famille des acides carboxyliques (de préférence l'acide citrique et ses sels), de préférence couplés à au moins une saponine.

Enfin dans un dernier aspect, la présente invention concerne une trousse de diagnostic moléculaire comprenant au moins un complexe tri-couches selon l'invention définie ci-dessus.

Cette dernière peut inclure également des réactifs permettant l'amplification spécifique des acides nucléiques susceptibles ou suspectés d'être contenus dans l'échantillon à tester, et/ou des réactifs permettant la détection et/ou la quantification des acides nucléiques susceptibles ou suspectés d'être contenus dans l'échantillon à tester

Ces trousses permettront de mettre en œuvre les différentes méthodes d'extraction et/ou de détection et/ou quantification décrites ci-dessus.

Les exemples et figures ci-joints représentent des modes de réalisation particuliers de l'invention et ne peuvent être considérés comme limitant la portée de la présente invention.

Figures :

Les différentes figures aident à la compréhension de l'invention et illustrent les résultats et performances obtenues avec le complexe selon l'invention.

- La figure 1 est un schéma du complexe selon l'invention dans une forme de réalisation possible, les hachures représentant le support solide, les étoiles représentant le au moins un composé magnétique, les cercles représentant ledit au moins un composé inorganique silicaté et la lettre L représentant ledit au moins un composé ayant une affinité pour ledit au moins un composé magnétique et/ou pour ledit au moins un composé inorganique silicaté.
- La figure 2 est un schéma de la formation du complexe selon l'invention dans une forme de réalisation préférée.
- La figure 3 correspond à une image au microscope électronique de complexes selon l'invention : particules de magnétite recouvertes quasiment entièrement de nanoparticules inorganiques silicatées Ludox® SM 7 nm et de pyrophosphate (invisible sur la figure).
- La figure 4 représente le rendement de capture (noir) et d'élution (quadrillé) de l'ADN pour des particules de magnétite recouvertes de composés inorganiques silicatés.

  - FSC 419 correspond aux résultats obtenus avec des particules de magnétite de 100nm ;
  - AL1001 correspond aux résultats obtenus avec des particules de magnétite de 100nm recouvertes de Ludox® 6% (poids/poids) à pH 3,5 ;
  - AL1018 correspond aux résultats obtenus avec des particules de magnétite de 100nm recouvertes de $MgSiO_4$ 50% (poids/poids) à pH 3,5 ;
  - AL 1024 correspond aux résultats obtenus avec des particules de magnétite recouvertes de $NaSiO_4$ 50% (poids/poids) à pH 3,5 ;
  - AL 1014 correspond aux résultats obtenus avec des particules de magnétite recouvertes de bentonite 30% (poids/poids) à pH 3,5.

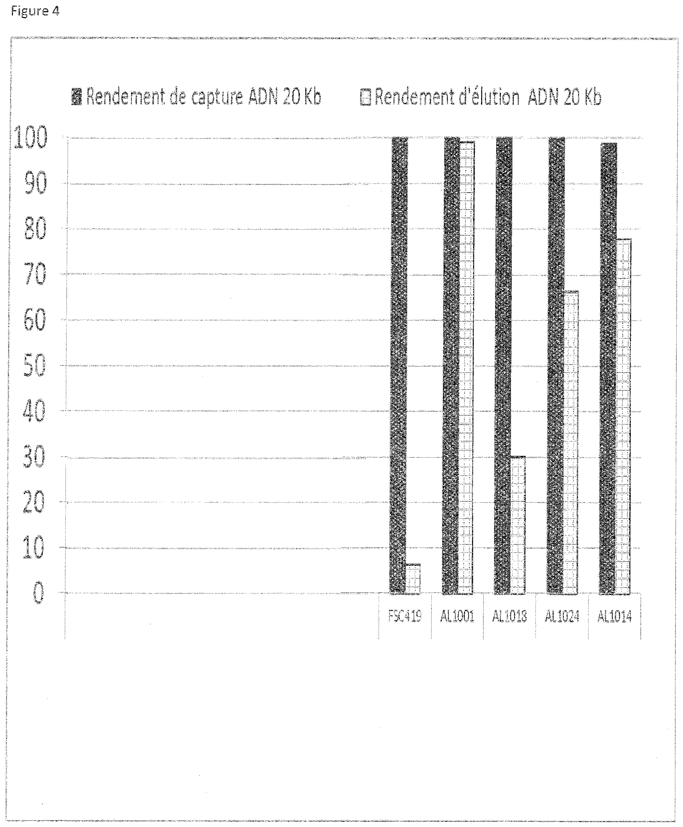

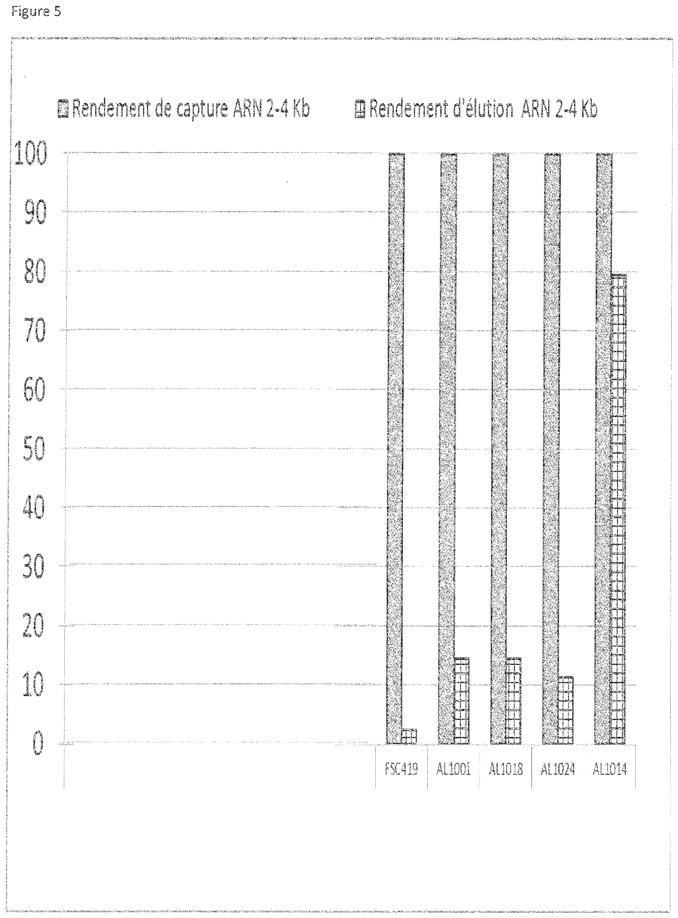

- La figure 5 représente le rendement de capture (gris) et d'élution (quadrillé) de l'ARN pour des particules de magnétite recouvertes de composés inorganiques silicatés. Les mêmes particules que celles de la figure 4 ont été testées.

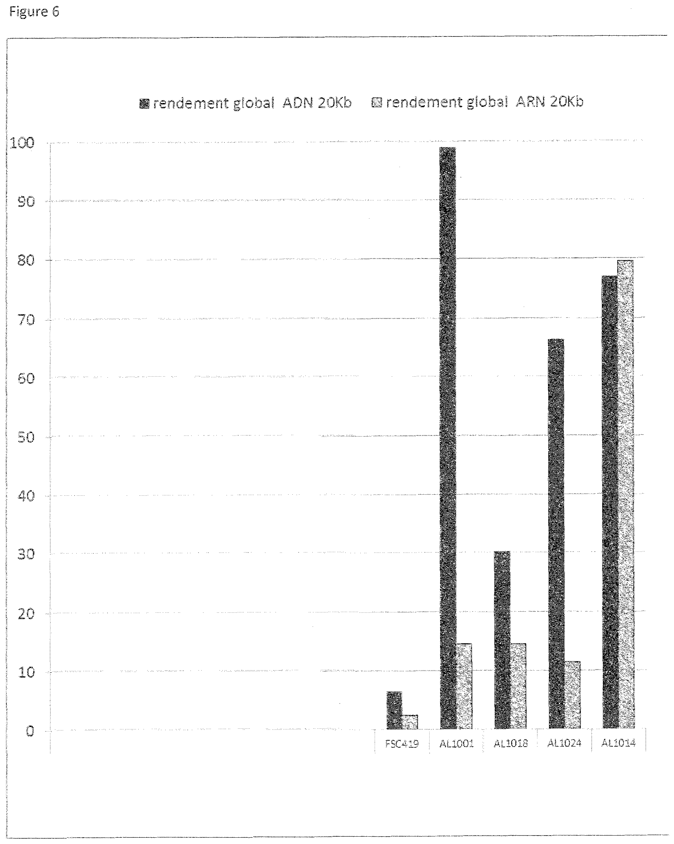

- La figure 6 représente le rendement global d'extraction de l'ADN (noir) et de l'ARN (gris) pour des particules de magnétite recouvertes de composés inorganiques silicatés décrites dans la figure 4.

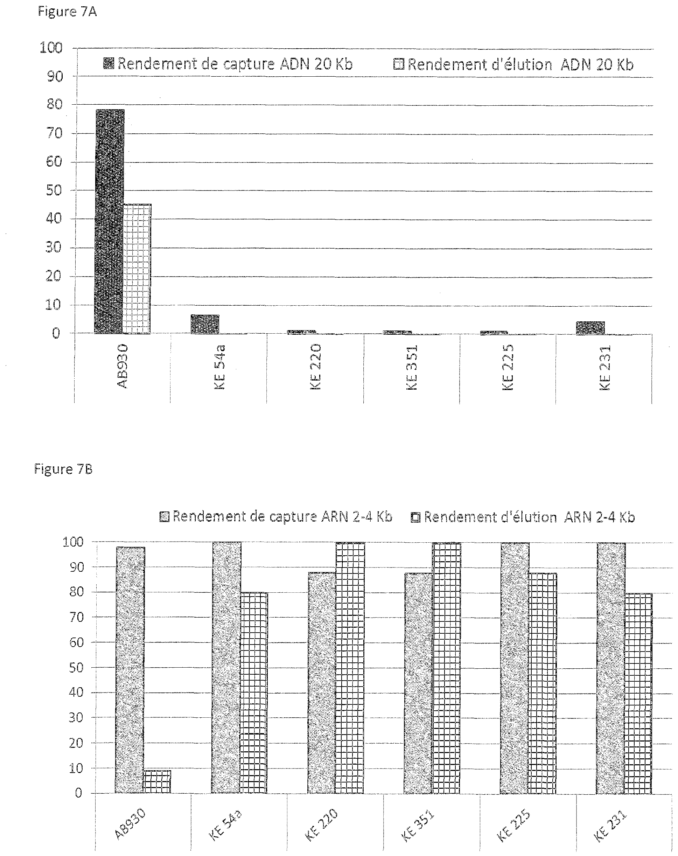

- La figure 7 A représente le rendement de capture (noir) et d'élution (quadrillé) de l'ADN et de l'ARN pour des particules de magnétite enrobées par un composé ayant une affinité pour ledit au moins un composé magnétique et/ou pour ledit au moins un composé inorganique silicaté.

- AB 930 correspond à des particules de magnétite seules ;
- KE54a correspond aux résultats obtenus avec des particules de magnétite recouvertes d'ions phosphates à 6% molaire. Par % molaire dans les figures et exemples, on entend ratio molaire Fer/ Composé ayant une affinité pour le composé inorganique silicaté et/ou le composé magnétique (que l'on peut appeler aussi ligand)
- KE220 correspond aux résultats obtenus avec des particules de magnétite recouvertes d'ions pyrophosphates à 6% molaire ;
- KE351 correspond aux résultats obtenus avec des particules de magnétite recouvertes d'ions triphosphates à 6% molaire ;
- KE225 correspond aux résultats obtenus avec des particules de magnétite recouvertes d'HEEPA acide(2-(2-(2-Hydroxyethoxy(ethoxy)ethyl-phosphonique) (Sikemia, Montpellier, France) à 10% molaire ;
- KE231 correspond aux résultats obtenus avec des particules de magnétite recouvertes de NTPA acide Nitrilotris (methylène) triphosphonique (Acros, Geel, Belgique) à 0,1% molaire.

- La figure 7 B représente le rendement de capture (gris) et d'élution (quadrillé) de l'ARN pour les mêmes particules que celles de la figures 7A.

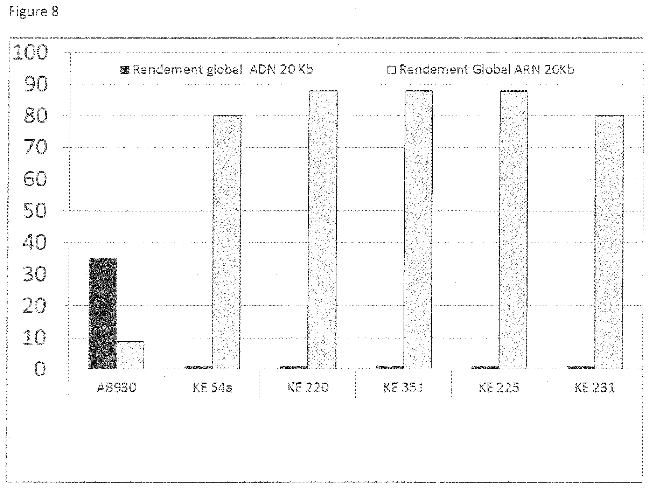

- La figure 8 représente le rendement global d'extraction de l'ADN (noir) et de l'ARN (gris) pour des particules des figures 7A et 7B.

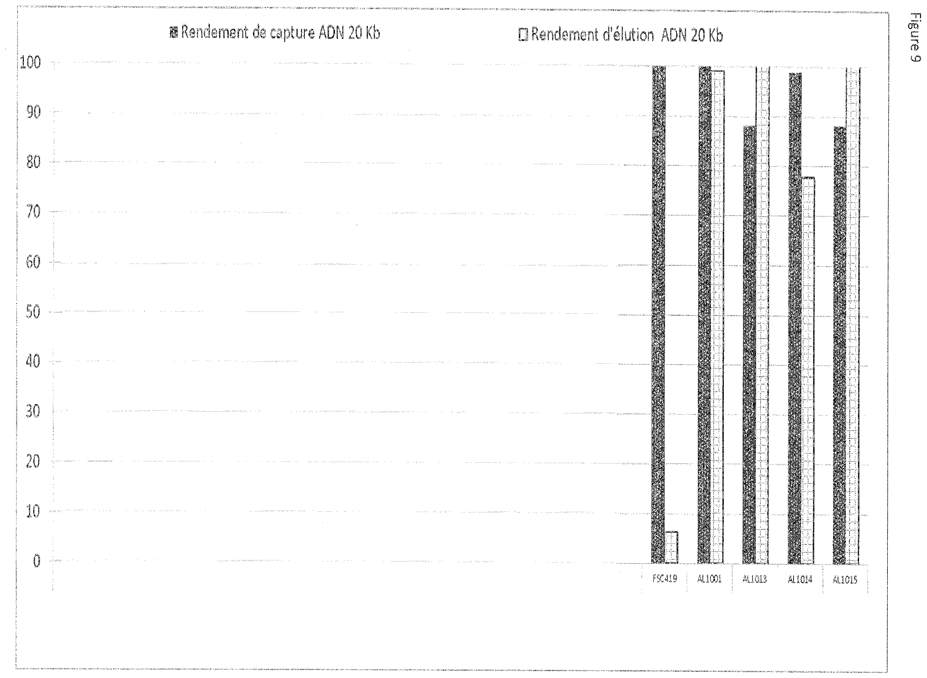

- La figure 9 représente le rendement de capture (noir) et d'élution (quadrillé) de l'ADN pour des particules de magnétite recouvertes de composés inorganiques silicatés et de composés ayant une affinité pour ledit au moins un composé magnétique et/ou pour ledit au moins un composé inorganique silicaté :

- FSC 419 correspond aux résultats obtenus avec des particules de magnétite de 100nm ;
- AL1001 correspond aux résultats obtenus avec des particules de magnétite de 100nm recouvertes de Ludox® 6% (poids/poids) à pH 3,5 ;
- AL1013 correspond aux résultats obtenus avec des particules de magnétite de 100nm recouvertes de Ludox® 6% (poids/poids) et d'ion pyrophosphate à 6,8% molaire ;
- AL 1014 correspond aux résultats obtenus avec des particules de magnétite recouvertes de bentonite 30% (poids/poids) à pH 3,5 ;
- AL1015 correspond aux résultats obtenus avec des particules de magnétite de 100nm recouvertes de bentonite 30% (poids/poids) et d'ion pyrophosphate à 6,8% molaire.

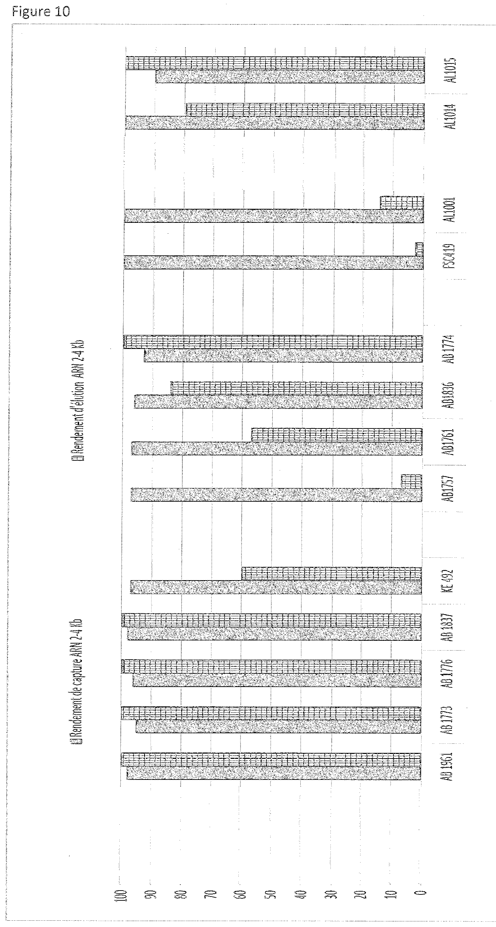

- La figure 10 représente le rendement de capture (gris) et d'élution (quadrillé) de l'ARN pour des particules de magnétite recouvertes de composés inorganiques silicatés et de composés ayant une affinité pour ledit au moins un composé magnétique et/ou pour ledit au moins un composé inorganique silicaté (en comparaison avec les références). Certaines particules de la figure 9 ont été testées vis-à-vis des ARN et les résultats figurent en figure 10. Les autres particules testées sont listées dans le tableau 3 de l'exemple 3 ci-dessous.

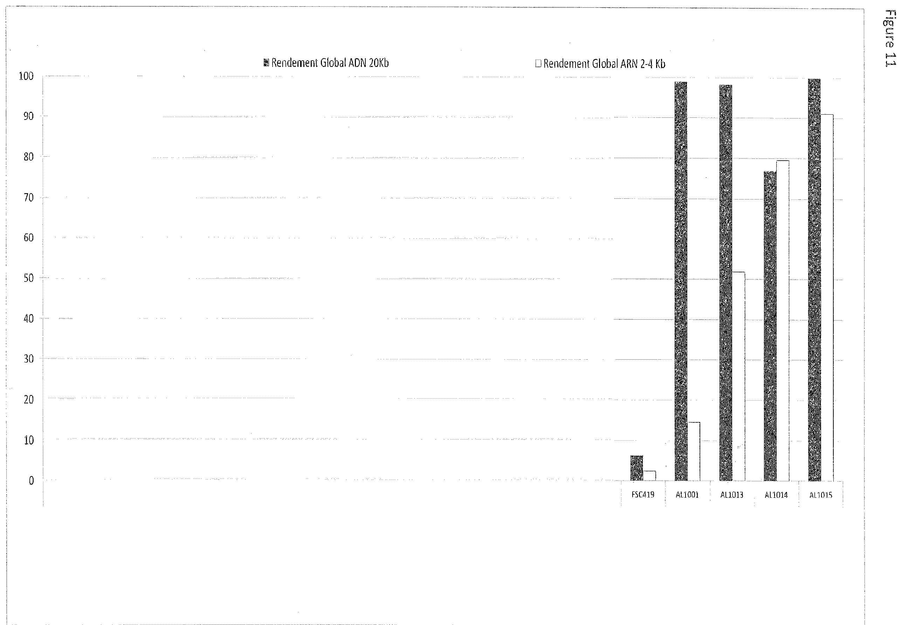

- La figure 11 représente le rendement global d'extraction de l'ADN (noir) et de l'ARN (gris) pour des particules de magnétite recouvertes de composés inorganiques silicatés et de composés ayant une affinité pour ledit au moins un composé magnétique et/ou pour ledit au moins un composé inorganique silicaté (en comparaison avec les références) des figures 9 et 10.

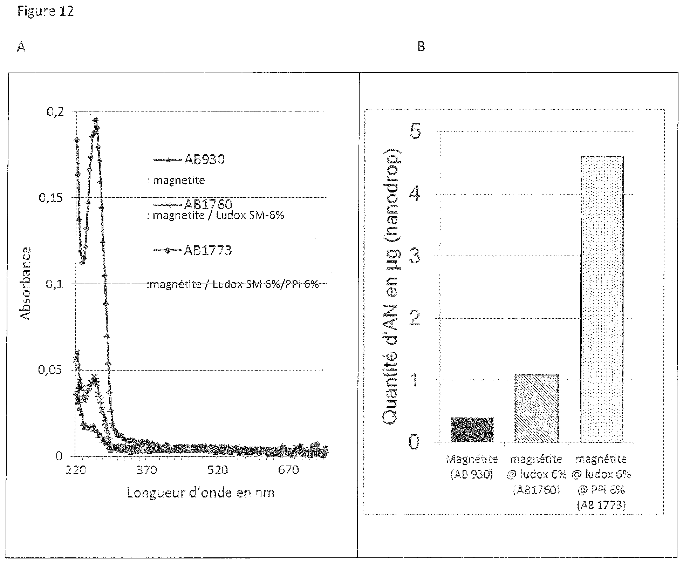

- La figure 12 représente l'efficacité d'extraction des acides nucléiques dans le sang avec des complexes tri-couches selon l'invention comprenant un type de magnétite (AB 930 de 100 nm) comme décrit dans l'invention (dosage UV au Nanodrop (ThermoScientific, Waltham, USA)).
- La figure 12 A représente l'absorbance de 3 types de particules en fonction de la longueur d'onde

- AB930 : magnétite pure - courbe avec les triangles
- AB1760 : magnétite recouverte de Ludox® SM 6% poids/poids - courbe avec les croix, et
- AB1773 : magnétite recouverte de Ludox® SM 6% puis d'ions pyrophosphate à 6,8% molaire-courbe avec les losanges.

- La figure 12B représente la quantité d'acides nucléiques capturés dans le sang en fonction de trois types différents de particules; les mêmes que celles de la figure 12A.

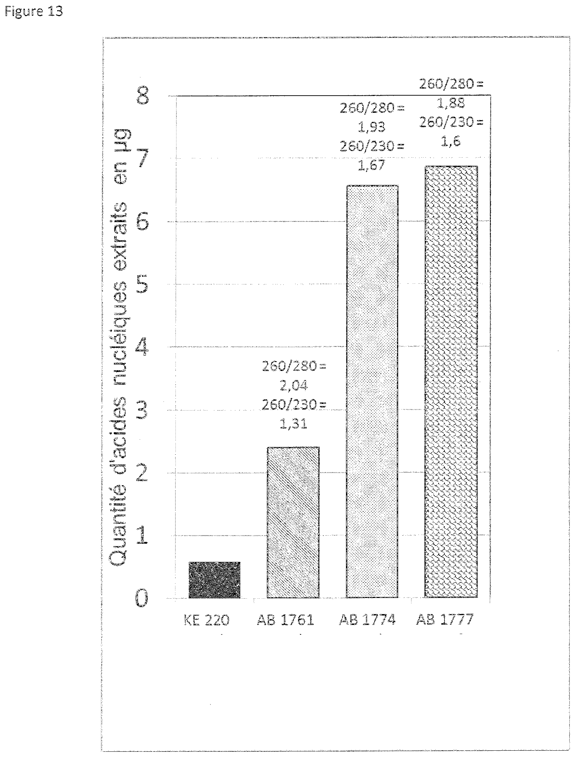

- La figure 13 illustre l'efficacité d'extraction des acides nucléiques dans le sang avec des complexes tri-couches selon l'invention comprenant un autre type de magnétite AB 1757 50 nm comme décrit dans l'invention (dosage UV au Nanodrop (ThermoScientific, Waltham, USA)). Les 4 types de particules testés sont les suivant :

- KE220 : magnétite enrobée d'ions pyrophosphates à 6,8% molaire ;
- AB1761 : magnétite enrobée de 6% poids/poids de Ludox® ;
- AB 1774 : magnétite enrobée de Ludox 6% poids/poids et d'ions pyrophosphates à 6,8% molaire, et
- AB1777 : magnétite enrobée de Ludox® à 6% poids/poids et d'ions triphosphates à 6,8% molaire.

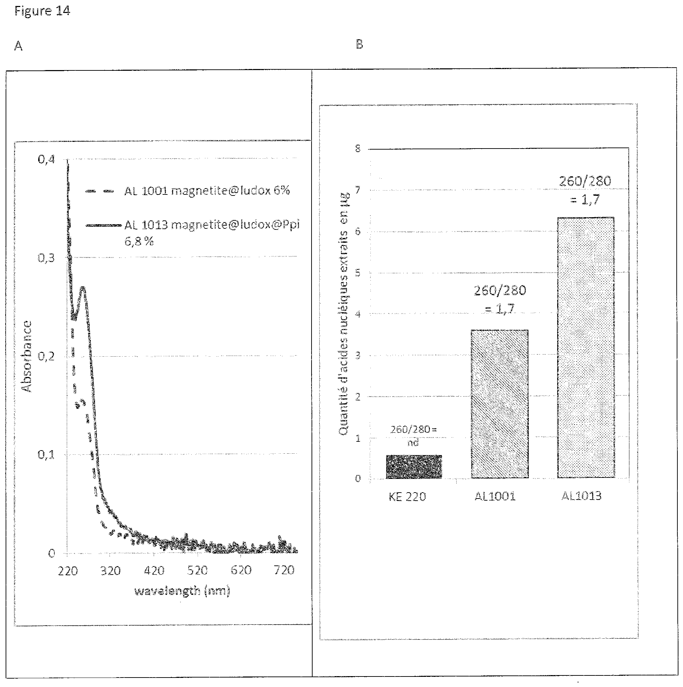

- La figure 14 illustre l'efficacité d'extraction des acides nucléiques dans le sang avec des complexes tri-couches selon l'invention comprenant un autre type de magnétite FSC 419 100 nm comme décrit dans l'invention (dosage UV au Nanodrop (ThermoScientific, Waltham, USA )).
- La figure 14 A représente l'absorbance de 2 types de particules en fonction de la longueur d'onde :

  - AL1001 : magnétite recouverte de Ludox® 6% poids/poids -courbe avec tirets, et
  - AL1013 : magnétite recouverte de Ludox® 6% puis d'ions pyrophosphate à 6,8% molaire -courbe pleine

- La figure 14B représente la quantité d'acides nucléiques capturés dans le sang en fonction de trois types différents de particules: KE220 : magnétite enrobée d'ions pyrophosphates à 6,8% molaire ; les 2 mêmes types de particules que celles de la figure 14A.

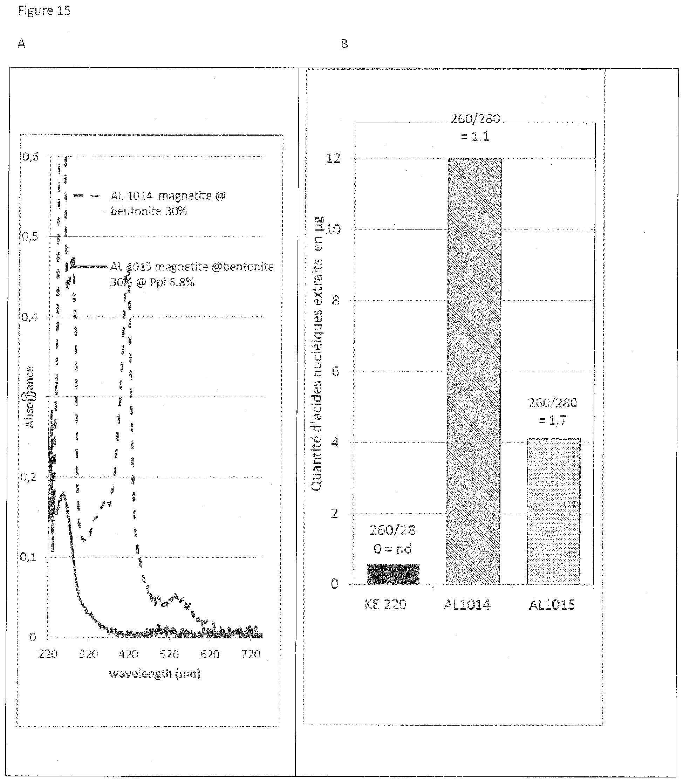

- La figure 15 illustre l'efficacité d'extraction des acides nucléiques dans le sang avec des complexes tri-couches selon l'invention comprenant un autre type de composé inorganique silicaté : la bentonite comme décrit dans l'invention (dosage UV au Nanodrop (ThermoScientific, Waltham, USA)).
- La figure 15 A représente l'absorbance de 2 types de particules en fonction de la longueur d'onde :

  - AL1014 : magnétite recouverte de bentonite 30% poids/poids -courbe avec tirets,
  - ÀLIOIS : magnétite recouverte de bentonite 30% puis d'ions pyrophosphate à 6,8% molaire-courbe pleine.

- La figure 15B représente la quantité d'acides nucléiques capturés dans le sang en fonction de trois types différents de particules : KE220 : magnétite enrobée d'ions pyrophosphates à 6,8% molaire ; les 2 mêmes types de particules que celles de la figure 15A.

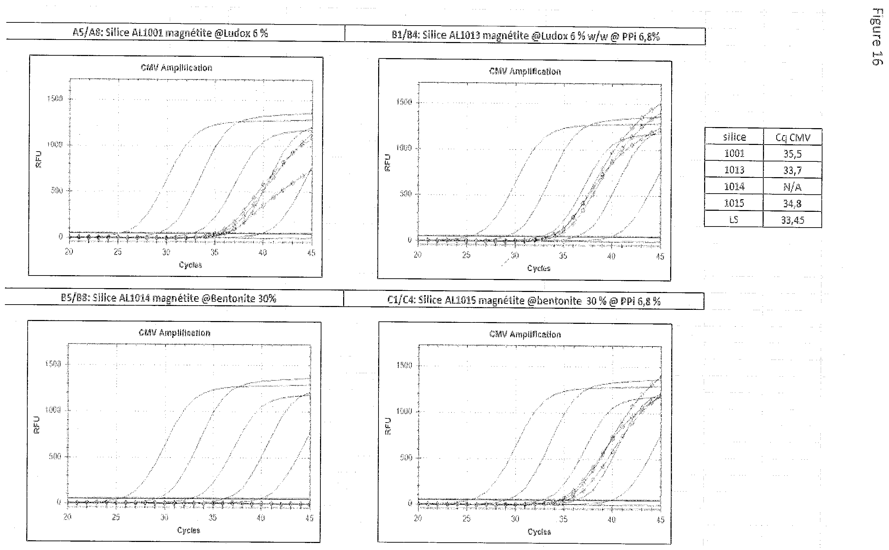

- La figure 16 illustre les résultats d'une amplification par PCR de l'ADN du virus CMV extrait par différent types différents de particules magnétiques simplement (complexes bi-couches) ou doublement enrobées (complexe tri-couches selon l'invention)

  - AL1001 : magnétite enrobée de Ludox 6% ;
  - AL1013 : magnétite enrobée de Ludox 6% poids/poids et d'ions pyrophosphates à 6,8% molaire;
  - AL1014 : magnétite enrobée de bentonite au ratio 30% poids/poids ;
  - AL1015 : magnétite enrobée de bentonite au ratio 30% poids/poids puis d'ions pyrophosphates à 6,8% molaire.

Exemples

**Exemple 1 : Enrobage de particules de magnétite par des composés inorganiques silicatés (magnétite/composés inorganiques silicatés)**

Des enrobages de particules de magnétite de 100nm environ, par des composés inorganiques silicatés commerciaux, ont été réalisés avec différents ratios magnétite/silice. Dans les exemples ci-dessous nous avons utilisé des Ludox SM®de 7 nm, de la bentonite, du silicate de magnésium ou de sodium, de la silice « fumée » ou du dioxyde de silicium.

L'enrobage est favorisé par des interactions électrostatiques opposées. Ainsi, ce dernier doit être réalisé dans une zone de pH où les nanoparticules inorganiques silicatées et la magnétite ont des charges de surface contraires.

Le point isoélectrique de la magnétite se situant à 6,8 et celui de la silice aux alentours de 2. L'enrobage doit être effectué à un pH compris entre 2 et 6,8 soit un pH retenu de 3,5 comme décrit ci-dessous.

**A- Protocole d'enrobage de la magnétite** :

1- De 1 à 400 mg de magnétite (11-814 $\mu$) à 92,1 mg/ml) sont lavés et repris dans le même volume avec une solution d'HCl à 1 mM pH 3 afin d'ajuster le pH de la suspension. Le volume de la solution de lavage dépend de la quantité de magnétite (entre 2 ml (1-75 mg de magnétite) et 10 ml (400 mg de magnétite)).

2- Les solutions de composés inorganiques silicatés indiqués ci-dessus sont préparées dans l'eau et leur pH est ajusté aux environs de 3 par addition de quelques gouttes de HCL à 1M avant d'ajuster leurs concentrations entre 30 et 300 mg/ml.

3- On mélange les suspensions de magnétite et de composés inorganiques silicatés dans les ratios indiqués dans le Tableau 1 ci-dessous et on mélange par vortex immédiatement avant d'ajuster le volume, avec une solution d'HCl à 1 mM pH 3, entre 2 ml et 10 ml selon la quantité de magnétite engagée. On trouvera dans le Tableau 1 quelques exemples d'enrobage de la magnétite par des nanoparticules inorganiques silicatées réalisés avec des ratios magnétite/silice (poids/poids) compris entre 1% et 50% afin d'obtenir des nanocomposites possédant des taux de recouvrement différents.

4- Le pH de la suspension finale est vérifié et doit être autour de 3-3,5 et selon le type de composé silicaté inorganique, on peut être amené à ajuster le pH avec quelques $\mu$l de NaOH ou de HCl à 1M.

5- La suspension est agitée sur un agitateur à rouleaux pendant 1 à 2 heures à température ambiante.

6- Un contrôle négatif peut être réalisé de la même manière mais avec une solution de soude à pH = 9-11 (100 $\mu$M - 1, 5 mM de NaOH) et sans ajuster le pH des composés inorganiques silicatés. A ce pH, lés nanoparticules de silice et de magnétite sont toutes les deux chargées négativement et la silice n'enrobe pas la magnétite. '

**B- Protocole de lavage de la suspension de particules magnetite/composés inorganiques silicatés**

1. Le surnageant est éliminé par aimantation des particules composites magnétite/nanoparticules silicatées nouvellement formées. La suspension est lavée d'abord à l'eau par aimantation puis à l'aide d'une solution de soude à 100 pM, pH 9 puis reprise dans la même solution et de façon facultative est soumise à des ultrasons pendant 10 min à 100% de la puissance (Vibra-cell 75042, Bioblock Scientific, Illkirch, France). La solution de soude est éliminée de la solution par aimantation. Ces étapes sont réalisées trois fois voire plus jusqu'à atteindre un pH de 9.

2. La solution de particules est lavée à l'eau par aimantation successives 3 fois jusqu'à pH 7 puis concentrée à 50 mg/ml. Un extrait sec est réalisé afin de vérifier cette concentration.

*Tableau 1* : *Détails expérimentaux du simple enrobage de la magnétite par des silicates*

| | Particule de magnétite porteuse | | Composés Inorganiques silicatés | | Ratio silice/magnétite (poids/poids) | pH Greffage | IEP , (Point Isoélectrique) |
|---|---|---|---|---|---|---|---|
| | Type de magnétite | Masse utilisée | Type de Nanoparticules | Masse utilisée | | | |
| AL 1001-CTRLE) | Magnétite FSC 419 100 nm 92.1 mg/ml. 814 $\mu$l | 75 mg | ludox SM 7 nm (Sigma Aldrich, ref. 420794) en solution dans l'eau à 30% à pH 9,5 | 15 $\mu$l (4,5mg) | 6% | 11 | 7.5 |
| KE 59 | Magnétite AB 930/ 100 nm | 75 mg | ludox SM 7 nm (Sigma Aldrich, référence 420794) en solution dans l'eau à 30% à pH 9,5 | 0.75 mg | 1% | 3-4 | 5.2 |
| KE 58 | Magnétite AB 930 100 nm | 75 mg | idem | 2.25 mg | 3% | 3-4 | 3.8 |
| AB 1760 | Magnétite AB 930 100 nm | 400 mg | idem | 29 mg | 6% | 3-4 | 2 |
| AL 1001 | Magnétite FSC 419 100 nm | 75 mg | idem | 15 $\mu$l (4,5mg) | 6% | 3-4 | 4 |
| AB 1761 | Magnétite AB 1757 50 nm | 400 mg | idem | 29 mg | 6% | 3-4 | nd |

(suite)

| | Particule de magnétite porteuse | | Composés Inorganiques silicatés | | Ratio silice/magnétite (poids/poids) | pH Greffage | IEP , (Point Isoélectrique) |
|---|---|---|---|---|---|---|---|
| | Type de magnétite | Masse utilisée | Type de Nanoparticules | Masse utilisée | | | |
| AL 1009 | Magnétite FSC 419 100 nm | 5 mg | bentonite (Aldrich 285234) 50 mg/ml dans l'eau à pH 10,4 | 6 $\mu$l (0,3mg) | 6% | 3-4 | <1 |
| AL. 1014 | Magnétite FSC 419 100 nm | 75 mg | bentonite (Aldrich 285234) 100 mg/ml dans l'eau ajustée à pH 7 avec HCL 1M | 318 $\mu$l (32 mg) | 30% | 3 | <1 |
| AL. 1018 | Magnétite FSC 419 100 nm | 75 mg | Magnésium trisilicate (Aldrich 63148) 150 mg/ml dans l'eau ajusté à pH 7 avec HCL 1M | 500 $\mu$l (75 mg) | 50% | 3 | 5.5 |
| AB 1992 | Magnétite AB 930 100 nm | 150mg | Magnésium trisilicate (Aldrich 63148) | 72mg | 50% | 3-4 | nd |
| AB 1024 | Magnétite AB 930 100 nm | 75 mg | Sodium trisilicate (Aldrich 63148) | 500 $\mu$l (75 mg) | 50% | 3 | 5.1 |
| AB 1959 | Magnétite AB 930 100 nm | 150 mg | Fumed silica (Aldrich S5130) | 18 mg | 10% | 3-4 | nd |
| AB 1960 | Magnétite AB 930 100 nm | 150 mg | Silicon dioxide (Aldrich 637238) | 18 mg | 10% | 3-4 | nd |

**C- Caractérisation physico-chimique par zetamétrie**

Les nanoparticules synthétisées sont analysées par zétamétrie sur l'appareil Zetasizer Nano ZS de Malvern Instruments (Malvern, Royaume Uni) afin de mesurer leur point isoélectrique. Les mesures se font à 0,3 mg/ml dans 3 solutions différentes, respectivement :

- HCl 5 mM, 10 mM NaCl pH 2,5 ;
- Tris, HCl 5 mM , 10 mM NaCl pH 7 ;
- et NaOH 5 mM , 10 mM NaCl pH 11,5.

Pour cela on prélève 6 $\mu$l de la suspension de particules à 50 mg/ml (0,3 mg) dans l'eau que l'on ajoute à 2 ml d'une des solutions ci-dessus contenues dans un flacon en polypropylène pour obtenir une suspension à 0,15 mg/ml. On s'assure par une mesure du pH que le pH de la solution n'est pas modifié par l'ajout des particules. La suspension de particules est alors introduite dans la cuve de mesure (Folded capillary cells, DTS 1061) à l'aide d'une seringue de 2 ml. Le programme de mesure choisi pour l'ensemble des mesures de potentiel Zéta est le programme « BMX Zêta » (les mesures sont effectuées à 21°C).

La mesure du potentiel zéta de ces particules en fonction du pH permet d'en déduire le point isoélectrique quand le potentiel zéta devient nul. Elle est indicative de l'état de la fonctionnalisation de surface de la magnétite.

Conclusion: D'une manière générale (Cf Tableau 1), on démontre un net décalage du point isoélectrique de la magnétite vers des valeurs plus basses correspondant bien à un revêtement par des composés inorganiques silicatés dont le niveau de revêtement peut être réglé par la concentration en composé inorganique silicaté(voir pour cela les expériences KE 59, KE 58 ou AB 1760 où le point iso-électrique évolue en fonction de la concentration en particules de composé inorganique silicaté Ludox®) ou AL 1018 et AL 1024 en fonction de la concentration en magnésium trisilicate ou sodium trisilicate.

**Exemple 2 : Enrobage de particules de magnétite par des composés ayant une affinité pour le au moins un composé magnétique et/ou pour le au moins un composé Inorganique silicaté. Pour plus de simplicité, ces derniers composés sont aussi appelés ligands (magnétite/ligand)**

Des particules de magnétite ont été enrobées de ligands comme décrit ci-après selon deux protocoles en fonction des pKa des composés ayant une affinité pour le au moins un composé magnétique et/ou pour le au moins un composé inorganique silicaté (ligands) choisis.

Comme pour l'adsorption des nanoparticules inorganiques silicatées, un ligand du fer s'adsorbera efficacement sur la magnétite dans un intervalle de pH où le ligand est chargé négativement et où la magnétite est chargée positivement soit à un pH compris entre le premier pKa du ligand et le point isoélectrique de la magnétite (6,8).

Suivant ce principe, les revêtements ou enrobages de composés ayant une affinité pour ledit au moins un composé magnétique et/ou pour ledit au moins un composé inorganique silicaté de type phosphates (pKa entre 1 et 12) et phosphonates (pKa entre 3 et 6) ont été réalisés avec respectivement un pH de la solution égal à pH 3 ou pH 5. Il est à noter que n'importe quel autre composé ayant une affinité pour ledit au moins un composé magnétique et/ou pour ledit au moins un composé inorganique silicaté peut être adsorbé (acides carboxyliques , etc.) suivant ce principe.

Les deux protocoles sont décrits ci-dessous.

**Protocole d'enrobage de la magnétite à pH=3 par des composés ayant une affinité pour le au moins un composé magnétique et/ou pour le au moins un composé inorganique silicaté de type phosphate**

1- Un volume minimal de magnétite de l'ordre de 550μl, correspondant à une masse de l'ordre de à 1 à 80 mg est lavé avec une solution d'HCl à 1mM pH 3 puis repris dans la même solution avant d'être introduit dans un micro tube en polypropylène de 1,5 ml.

2- Le ligand de type phosphate en solution dans l'eau à 40 mM dont le pH a été ajusté à pH 3 est ajouté sous vortex pour correspondre à une fraction molaire comprise entre 0,1% à 10% molaire par rapport au fer. Ex:

$$\text{Fraction molaire en ligand par rapport au fer} = \frac{nb\ mole\ de\ ligand}{(nb\ mole\ ligand) + (nb\ mole\ Fe3O4\ x3)}$$

$$= \frac{V_{ligand} \times [ligand]}{(V_{ligand} \times [ligand]) + (\frac{masse\ Fe3O4}{M(Fe3O4)} x3)}$$

3- Le pH est contrôlé à l'aide d'un pHmètre, il doit être de l'ordre de 3. Il n'est en général pas nécessaire de réajuster le pH mais on peut le faire si besoin avec quelques μl de NaOH ou de HCl à 0,1M.

4- On complète à 1,5 ml avec une solution d' HCl à 1mM pH 3.

5- Le tube eppendorf est agité sur rouleaux pendant 2 heures à température ambiante.

6- Le milieu réactionnel est ensuite lavé à l'eau par aimantation jusqu'à l'obtention d'un pH de 7.

7- Les particules sont reprises dans la quantité adéquate d'eau afin d'obtenir une concentration de 50 mg/ml (vérifié par mesure de l'extrait sec).

**Protocole d'enrobage de la magnétite à pH=5 par des composés ayant une affinité pour ledit au moins un composé magnétique et/ou pour ledit au moins un composé inorganique silicaté de type phosphonates**

1- Un volume minimal de magnétite de l'ordre de 550μl, correspondant à une masse de l'ordre de 30-80 mg est lavé avec une solution d'HCl à 10μM pH 5 puis repris dans la même solution avant d'être introduit dans un tube eppendorf de 1,5 ml.

2- Le ligand en solution dans l'eau à 40 mM dont le pH a été ajusté à pH 5 est ajouté sous vortex pour correspondre à une fraction molaire compris entre 0,1% à 10% molaire par rapport au fer.

3- Le pH est contrôlé à l'aide d'un pHmètre puis ajusté aux alentours de 5 si nécessaire par une solution de soude ou d'HCl à 0.1M.

4- On complète à 1,5 ml avec une solution d'HCl à 10μM pH 5.

5- Le mélange réactionnel est agité au thermomixeur pendant 12 heures à 60°C.

6- Les particules sont ensuite lavées à l'eau par aimantation jusqu'à l'obtention d'un pH de 7.

7- Les nanoparticules sont reprises dans la quantité adéquate d'eau afin d'obtenir une concentration de 50 mg/ml (vérifié par mesure de l'extrait sec).

On trouvera dans le tableau ci-dessous un récapitulatif de quelques exemples d'enrobage de la magnétite par des composés ayant une affinité pour ledit au moins un composé magnétique et/ou pour ledit au moins un composé inorganique silicaté (à concentration optimale en composé ligand pour satisfaire aux meilleures conditions d'extraction des acides nucléiques).

*Tableau 2 : Détail expérimental du simple enrobage de la magnétite par des composés ayant une affinité pour le au moins un composé magnétique et/ou pour le au moins un composé inorganique silicaté*

| | Particule de magnétite porteuse | | Composés ayant une affinité pour ledit au moins un composé magnétique et/ou pour ledit au moins un composé Inorganique silicaté (ligand) | | | | |
|---|---|---|---|---|---|---|---|
| | Type de magnétite | Masse utilisée | Type de ligand | Volume Utilisé à 40 mM | Ratio molaire Fe/ ligand | pH Greffage | IEP |
| KE 54a | Magnétite AB 930 100 nm | 80 mg | Acide orthophosphorique (Aldrich ref. : 345245 ) | 1900 µl | 6,8% | 3,5 | 5.2 |
| KE 220 | idem | 80 mg | Pyrophosphate de sodium (Aldrich ref. : 221368 ) | 1900 µl | 6,8% | 3,5 | <2 |
| KE 351 | idem | 80 mg | Triphosphate de sodium (Aldrich ref. : 72061 ) | 1900 µl | 6,8% | 3,5 | 3.2 |
| KE 225 | Idem | 80 mg | HEEEPA=2-(2-(2-Hydroxyethoxy(ethoxy)ethyl-phosphonic acid (Sikemia, Montpellier, France) | 1900 µl | 10% | 5 | 4.3 |
| KE 231 | Idem | 80 mg | NTPA Nitrilotris(methylène) triphosphonic acide (Acros, Geel, Belgique) | 1900 µl | 0,1% | 5 | nd |

Conclusion: On constate un net déplacement de la valeur du point isoélectrique vers des valeurs plus basses que celle de la magnétite (point isoélectrique IEP: 6-7) ce qui démontre l'enrobage de la magnétite et la modification de sa charge de surface et donc de ses propriétés de surface.

**Exemple 3: Enrobage par des composés ayant une affinité pour le au moins un composé magnétique et/ou pour le au moins un composé inorganique silicaté de particules de magnétite préalablement revêtues par des composés inorganiques silicatés (magnétite/composés Inorganiques silicatés/ligands)**

Les particules composites magnétite/composés inorganiques silicatés obtenues dans l'exemple 1 sont à leur tour enrobées de composés ayant une affinité pour le au moins un composé magnétique et/ou pour le au moins un composé inorganique silicaté exactement comme décrit dans l'exemple 2 sauf que ce sont des particules magnétite/composés inorganiques silicatés qui sont utilisées au lieu de la magnétite seule (Cf. Tableau 3). Comme dans l'exemple 2, deux protocoles de revêtements sont utilisés selon le pKa du composé ayant une affinité pour le au moins un composé magnétique et/ou pour le au moins un composé inorganique silicaté (ligand). Des mesures de point isoélectriques sont faites sur ces particules.

*Tableau 3: Détail expérimental de l'enrobage de la magnétite/composés inorganiques silicatés par des composés ayant une affinité pour le au moins un composé magnétique et/ou pour le au moins un composé inorganique silicaté*

| | Particule de magnétite porteuse | | Composés ayant une affinité pour ledit au moins un composé magnétique et/ou pour ledit au moins un composé inorganique silicaté (ligand) | | | | |
| --- | --- | --- | --- | --- | --- | --- | --- |
| | Type de magnétite | Masse utilisée | Type de ligand | Volume Utilisé à 40 mM | Ratio molaire Fe/ ligand | pH Greffage | Pot Zeta à pH 7 |
| AB1961 | Magnétite 100 nm/ Ludox ® 6% (AB 1760) | 30 mg | Pi = ion phosphate | 710 μl | 6.8% | 3.5 | nd |
| AB 1773 | idem | 30 mg | PPi = ion pyrophosphate | 710 μl | 6.8% | 3.5 | nd |
| AB 1776 | idem | 30 mg | PPPi = ion triphosphate | 710 μl | 6.8% | 3.5 | nd |
| KE 492 | idem | 30 mg | HEEEPA = 2-(2-(2-Hydroxyethoxy{ethoxy)e thylphosphonic acid)) (Sikemia, Montpellier, France) | 1420 μl | 10% | 5.6 | nd |
| AB1837 | idem | 30 mg | NTPA Nitrllotris (methylène) triphosphonic acid (Acros, Geel, Belgique) | 14.2 μl | 0,1 % | 5.3 | |
| AL 1013 | Magnétite 100 nm / Ludox ® 6% (AL 1001) | 30 mg | PPi | 875 μl | 8.3% | 3 | 1.5 |
| AB 1774 | Magnétite 50 nm / Ludox ® 6% (AB 1761) | 30 mg | PPi | 710 μl | 6.8% | 3.6 | nd |
| AB 1836 | Idem | 30 mg | HEEPA | 1420 μl | 10% | 5.6 | nd |
| AB1777 | idem | 30 mg | PPPi | 710 μl | 6.8% | 3.2 | nd |
| AL 1015 | Magnétite 100 nm / bentonite 30% (AL ' 1014) | 37 mg | PPi | 1080 μl | 8.3% | 3 | <1 |

Conclusion: la mesure du point isoélectrique montre que l'enrobage par ledit au moins un composé ayant une affinité pour ledit au moins un composé magnétique et/ou ledit au moins un composé inorganique silicaté ne modifie guère la charge de surface des particules. Ce qui démontre que les particules restent négativement chargées à pH 7.

**Exemple 4: Mesures des rendements d'extraction de l'ADN et de l'ARN avec des particules magnétiques enrobées de composés Inorganiques silicatés décrites à l'exemple 1**

Afin de mesurer les capacités et les performances des particules magnétiques enrobées de composés inorganiques silicatés, décrites dans l'exemple 1, à extraire des acides nucléiques, nous avons utilisé le protocole décrit ci-dessous avec les acides nucléiques décrit dans le Tableau 4.

*Tableau 4: Acides nucléiques utilisés dans le test d'extraction des acides nucléiques*

| Acide Nucléique | Nom du fournisseur | Référence | Taille moyenne |
|---|---|---|---|
| ADN de testicules de saumon | Sigma, St Louis, USA | D1626-1G | 20 Kb |
| ADN de placenta humain | Fluka, Buchs, Suisse | 31167 | 20 Kb |
| ARN humain de cellules MRC2 | R&D Biotech , Besançon, France | | 2-4 Kb |

**Mesure du rendement de capture**

1- On prépare dans un micro tube en polypropylène de 1,5 ml la solution suivante:

a- 100 μl de chlorure de guanidinium (GuHCl) 8M tamponné avec du Tris HCl à 50 mM pH 7 (pH rigoureusement remesuré avant la manipulation)
b- 30 μl de Tris HCl à 100 mM pH 7
c- 20 μl (20 μg) de la solution d'acides nucléiques à 1 mg /ml dans 50 mM Tris HCl pH 7 (dans le cas de l'ADN) ou à 1 mg /ml dans l'eau dans le cas de l'ARN.
d- On mélange par vortex.

2- On ajoute extemporanément 50 μl des particules préparées dans l' exemple 1 décrit précédemment à 50 mg/ml dans l'eau (2.5 mg). Les conditions finales sont: 100 μg /ml d'acides nucléiques pour 2.5 mg de particules dans 200 μl de GuHCl 4M, Tris HCL 45 mM pH 7).
3- On laisse agiter sur un agitateur (Thermomixer, Eppendorf, Le Pecq, France ,) à 1000 rpm à 25°C pendant 15 min.
4- On fait une mesure de le densité optique DO à 260nm.
5- On prélève, après aimantation, 1.7 μl du surnageant de la suspension que l'on dose par spectrophotométrie UV à 260 nm à l'aide d'un Nanodrop (ThermoScientific, Waltham, USA) afin d'en déterminer la concentration en acide nucléiques.
6- On compare la concentration en acides nucléiques de la solution à celle d'une solution de référence, solution de départ, réalisée sans particules afin de mesurer le rendement de capture :

$$Rendement\ de\ capture\ \text{à}\ 15\ min = \frac{DO260nm\ initiale - DO260\ nm\ résiduelle\ (15\ min)}{DO260nm\ initiale}$$

**Mesure du rendement d'élution**

Les particules, sur lesquelles sont immobilisés les acides nucléiques, sont alors lavées par aimantation.

1- On aimante la suspension préparée précédemment et on jette le surnageant.
2- On ajoute 185 μl d'une solution de GuHCl 4M, Tris HCL 50 mM pH 7. On mélange par vortex, on aimante et on élimine le surnageant en s'assurant par dosage UV au Nanodrop (ThermoScientific, Waltham, USA) qu'il ne contient pas d'acides nucléiques.
3- On ajoute 185 μl du tampon NucliSENS easyMAG Extraction Buffer 2 (Ref BMX 280131, bioMérieux, Marcy l'étoile, France). On mélange par vortex, on aimante et on élimine le surnageant en s'assurant par dosage UV au Nanodrop (ThermoScientific, Waltham, USA) qu'il ne contient pas d'acides nucléiques.
4- On ajoute 185 μl du tampon NucliSENS easyMAG Extraction Buffer 3 (ref BMX 280132, bioMérieux, Marcy l'étoile, France), on mélange par vortex, aimante et élimine le surnageant en s'assurant par dosage UV au Nanodrop (ThermoScientific, Waltham, USA) qu'il ne contient pas d'acides nucléiques.
5- On ajoute 185 μl du tampon NucliSENS easyMAG Extraction Buffer 3 (ref BMX 280132, bioMérieux, Marcy l'étoile, France), on mélange par vortex et on incube à 70°C à 1000 rpm pendant 10 min.
6- On aimante et mesure la concentration en acides nucléiques au Nanodrop (ThermoScientific, Waltham, USA) à 260 nm afin de calculer le rendement d'élution.

$$Rendement\ d'élution\ à\ 15\ min = \frac{DO260\ nm\ après\ 15\ min\ d'élution}{DO260nm\ initiale - DO260\ nm\ résiduelle\ (15\ min)}$$

Le protocole décrit ci-dessus permet de mesurer un rendement de capture et un rendement d'élution, basé sur ce qui a été capturé, comme représenté sur les figures 4 et 5. On démontre ainsi que, par rapport à la magnétite, la capture de l'ADN n'est que peu modifiée par le revêtement de composés inorganiques silicatés mais que son l'élution est améliorée de façon remarquable. Par contre, on n'a pas de modification de la capture de l'ARN et le revêtement de composé inorganique silicaté apporte également une aide à son élution (en moindre mesure). Ce phénomène peut être généralisé à divers types de composés inorganiques silicatés comme représenté sur les figures.

Nous avons, pour plus de simplicité, représenté sur la figure 6 le rendement global d'extraction en ADN ou en ARN (multiplication du rendement de capture par le rendement d'élution comme indiqué sur les figures 4 et 5). Ainsi, le simple enrobage de la magnétite par des composés inorganiques silicatés et suivant le protocole décrit ci-dessus constitue donc en lui-même une amélioration significative des propriétés d'extraction des acides nucléiques de la magnétite.

**Exemple 5: Rendement d'extraction de l'ADN et de l'ARN avec des particules magnétiques enrobées par des composés ayant une affinité pour ledit au moins un composé magnétique et/ou pour ledit au moins un composé inorganique silicaté décrites à l'exemple 2**

Des expériences d'extraction de l'ADN et de l'ARN ont été réalisées en suivant le protocole décrit dans l'exemple 4 en utilisant des particules de magnétite enrobées seulement de composé ayant une affinité pour le au moins un composé magnétique et/ou pour le au moins un composé inorganique silicaté comme synthétisées à l'exemple 2. La figure 7 (figure 7A et 7B) en décrit les mesures de rendement de capture et de rendement d'élution, On démontre que, par rapport à la magnétite, la capture de l'ADN est totalement inhibée (figure 7A) alors que la capture de l'ARN n'est pas modifiée mais que son élution est améliorée de façon remarquable (figure 7B).

Nous avons également, pour plus de simplicité, représenté sur la figure 8 le rendement global d'extraction en ADN ou en ARN (multiplication du rendement de capture par le rendement d'élution) qui démontre à nouveau et toujours en comparaison avec la magnétite, que l'adsorption ou capture de composés ayant une affinité pour ledit au moins un composé magnétique et/ou pour ledit au moins un composé inorganique silicaté sur de la magnétite améliore de façon spectaculaire le rendement d'extraction de l'ARN alors qu'il réduit à néant celui de l'ADN.

**Exemple 6: Rendement d'extraction de l'ADN et de l'ARN avec des particules magnétiques doublement enrobées décrites à l'exemple 3**

Des expériences d'extraction de l'ADN et de l'ARN ont été réalisées en suivant le protocole décrit dans l'exemple 4 en utilisant des particules magnétiques doublement revêtues : 1) de au moins un composé inorganique silicaté et 2) de au moins un composé ayant une affinité pour ledit au moins un composé magnétique et/ou pour ledit au moins un composé inorganique silicaté comme synthétisées à l'exemple 3. Nous avons utilisé deux lots de magnétite, l'un composé de particules de 50 nm et l'autre de 100 nm. A notre grande surprise, on constate que les particules doublement enrobées donnent d'excellents résultats (représente le représentés sur les figures 9 et 10) et qu'elles permettent d'extraire beaucoup mieux l'ADN et l'ARN que celles qui sont enrobées une fois (particules de l'exemple 1 ou 2), les résultats d'extraction sont bien meilleurs que la simple addition des deux résultats d'extraction avec les complexes bi-couches des exemples 1 et 2, à savoir les particules magnétiques enrobées avec le composé inorganique silicaté (exemple 1) et les particules revêtues de composés ayant une affinité pour le composé inorganique silicaté et/ou pour le composé magnétique (exemple 2). La capacité de capture de l'ADN est sensiblement maintenue par rapport à la magnétite mais l'élution est facilitée. En ce qui concerne l'ARN la capacité de capture est également maintenue de façon surprenante et l'élution très nettement favorisée.

Au bilan et comme cela est résumé sur la figure 11, ces résultats sont remarquables et montrent que les complexes de l'invention offrent un double niveau de performances : l'enrobage avec des composés inorganiques silicatés permet de conserver le bénéfice d'un bon rendement d'extraction de l'ADN tout en augmentant de façon spectaculaire le rendement d'extraction de l'ARN suite à l'adsorption des composés ayant une affinité pour le composé inorganique silicaté et/ou pour le composé magnétique.

Il est notable de remarquer que l'enrobage de la magnétite par un composé ayant une affinité pour le composé inorganique silicaté et/ou pour le composé magnétique, quand des composés inorganiques silicatés ont été adsorbés préalablement, n'abaisse pas le rendement d'extraction de l'ADN alors que l'on aurait pu s'attendre à cette baisse puisque c'est le cas quand seuls sont adsorbés les composés ayant une affinité pour le composé inorganique silicaté et/ou pour le composé magnétique. De plus, on observe, de façon nette, que ce double enrobage offre un effet synergique et coopératif dans l'extraction des acides nucléiques car il donne de meilleurs résultats que l'addition des résultats obtenus avec un simple enrobage qu'il soit avec des composés inorganiques silicatés ou avec des composés ayant une affinité pour le composé inorganique silicaté et/ou pour le composé magnétique.

**Exemple 7 : Démonstration de l'efficacité des particules doublement enrobées (complexe tri-couches) pour l'extraction des acides nucléiques contenus dans un échantillon de sang en utilisant des particules magnétiques doublement enrobées telles que décrites dans l'exemple 3**

L'automate d'extraction des acides nucléiques easyMAG® (bioMérieux, Marcy l'Etoile, France) a été utilisé pour tester les particules magnétiques doublement enrobées (complexe tri-couches) préparées à l'exemple 3 quant à leurs capacités à extraire des acides nucléiques contenus dans les cellules blanches d'un échantillon de sang. Pour cela, le protocole ci-dessous a été utilisé :

1- 200$\mu$l de sang sont ajoutés à 2 ml de tampon de lyse (NucliSENS lysis buffer BMX 200292, bioMérieux, Marcy l'Etoile, France)
2- L'échantillon est soumis à un mélange par vortex pendant quelques secondes puis laissé au repos à température ambiante pendant 10 minutes.
3- On ajoute 50 $\mu$l de particules magnétiques à 50 mg/ml dans de l'eau à la solution préparée dans l'exemple 3 soit 2,5 mg et on mélange par vortex immédiatement pendant 5 secondes.
4- La suspension est incubée au repos pendant 10 min sans agitation avant d'être transférée dans une navette easyMAG® où les particules magnétiques vont être lavées avant d'en éluer les acides nucléiques,

a. Brièvement, les particules sont lavées dans l'automate avec quelques ml de tampon NucliSENS easyMAG Extraction Buffer 1 (Ref BMX 280130, bioMérieux, Marcy l'étoile, France))
b. Puis reprises avec quelques ml d'une solution d'éthanol à 70 %
c. L'élution se fait finalement avec 50 $\mu$l de tampon NucliSENS easyMAG Extraction Buffer 3 (ref BMX 280132, bioMérieux, Marcy l'étoile, France) à 70°C pendant 5 minutes.

5- La qualité des acides nucléiques extraits est ensuite évaluée par mesure de l'absorption de l'éluat entre 220 et 750 nm (Nanodrop, Thermo Fischer Scientific, USA )

**Exemple 7A** :

On démontre sur les spectrophotogrammes ci-dessous donnés en figure 12 que la qualité et la quantité des acides nucléiques extraits croissent avec le complexe tri-couches (aussi appelées particules ayant un double enrobage) de façon remarquable. La magnétite (AB 930, 100 nm) n'extrait presque aucun acide nucléique, la magnétite/Ludox ® 6 % (AB1760) extrait 3 fois plus des acides nucléiques (ratio 260/280 =1.9) avec cependant un ratio 260/230 de 1.1 qui indique une contamination en sels. La magnétite/Ludox ® 6 %/PPi 6.8% (AB 1773) extrait à nouveau 4 fois plus, d'acides nucléiques mais cette fois d'excellente qualité puisque le ratio 260/280 étant égal à 2.1 et le ratio 260/230 étant égal à 1.7.
Conclusion : Ceci démontre l'effet bénéfique et coopératif du complexe tri-couches sur l'extraction des acides nucléiques à partir d'un échantillon complexe tel que le sang.

**Exemple 7B** :

Un autre lot de magnétite (AB 1757, 50 nm) a été également enrobé par du pyrophosphate (= KE220) ou des nano particules silicatées Ludox® à hauteur de 6% (= AB 1761). Le produit AB1761 a ensuite été enrobé avec 6.8% de PPi (AB 1774) ou 6.8 % de PPPi (AB 1777). Ces particules ont ensuite été utilisées pour extraire des acides nucléiques d'un échantillon sanguin comme décrit précédemment. Les résultats de la figure 13 montrent à nouveau clairement l'augmentation de l'efficacité de capture et d'élution des acides nucléiques avec des puretés 260/280 remarquables. On peut remarquer également que la capacité d'extraction des acides nucléiques est plus importante car les particules sont plus petites. On peut noter aussi que la magnétite recouverte seulement de ligands du fer (KE220) donne de très mauvais résultats d'extraction montrant de façon encore plus claire, l'effet coopératif des deux types de revêtement (composé inorganique silicaté + composé ayant une affinité pour le au moins un composé magnétique et/ou le au moins un composé inorganique silicaté)
Conclusion : On montre bien avec cet exemple qu'il y a un effet synergique des deux revêtements sur la particule magnétique (complexe tri-couches selon l'invention) et ceci pour l'extraction des acides nucléiques à partir du sang. De plus, on montre que plusieurs types de complexes selon l'invention peuvent donner des résultats très performants.

**Exemple 7C :**

Un autre lot de magnétite (FSC 419, 50 nm) a été également enrobé par des nano particules silicatées Ludox® à hauteur de 6% (= AL 1001). Le produit AL 1001 a été ensuite enrobé avec 6,8% de PPi 6.8 % (AL 1013). Ces particules ont ensuite été utilisées pour extraire des acides nucléiques d'un échantillon sanguin comme décrit précédemment. Les résultats de la figure 14 montrent à nouveau clairement l'augmentation de l'efficacité de ces particules selon l'invention dans l'extraction des acides nucléiques avec des puretés 260/280 remarquables. Il est encore clair que l'extraction des acides nucléiques est potentialisée lorsque le complexe est tri-couches. En effet, avec des complexes bi-couches (magnétite/PPi ou magnétite/Ludox), l'extraction est nettement moins bonne. Il n'était pas du tout prévisible que le complexe tri-couches selon l'invention donnerait des résultats d'extraction encore meilleurs que l'addition des résultats obtenus pour chacun des complexes bi-couches.

**Exemple 7D :**

Le même lot de magnétite (FSC 419, 50 nm) a été également enrobé par des nano particules silicatées de bentonite à hauteur de 30% ( = AL 1014). Le produit AL 1014 a été ensuite enrobé avec 6.8% de PPi (AL 1015). Ces particules ont ensuite été utilisées pour extraire des acides nucléiques d'un échantillon sanguin comme décrit précédemment. Les résultats de la figure 15 montrent à nouveau clairement l'augmentation de l'efficacité des particules de l'invention (complexe tri-couches) dans l'extraction des acides nucléiques avec des puretés 260/280 remarquables. Il est notable de remarquer que les particules AL 1014 simplement recouvertes de bentonite ne sont pas efficaces pour extraire les acides nucléiques avec une bonne pureté (ratio 260/280 = 1,1 seulement, ce qui indique une très forte contamination en protéines comme visualisé sur le spectre UV de la figure 15). Par contre, le revêtement de ces particules (complexe bi-couches) par du pyrophosphate PPi améliore l'extraction de façon considérable (ratio 260/280 = 1,7), ce qui montre à nouveau le phénomène de coopération et synergie apporté par le double enrobage pour former le complexe tri-couches de l'invention.

**Exemple 8 : Amplification et détection par PCR en temps réel d'acides nucléiques du cytomegalo virus (CMV) extraits d'un échantillon de sang par des complexes tri-couches selon l'invention (particules telles que décrites dans l'exemple 3).**

L'automate d'extraction des acides nucléiques easyMAG® (bioMérieux, Marcy l'Etoile, France) ainsi que le kit de quantification par PCR en temps réel CMV R-gene (bioMérieux, Marcy l'Etoile, France) ont été utilisés pour tester les complexes tri-couches selon l'invention (particules magnétiques doublement enrobées préparées à l'exemple 3) quant à leurs capacités à extraire des acides nucléiques à partir d'un échantillon de sang et à la capacité de ces acides nucléiques extraits à être ensuite amplifiés. Pour cela le protocole ci-dessous a été utilisé :

1. 200μl de sang contaminé par l'ajout d'une culture virale de Cyto Megalo Virus (CMV produit à partir de CMV Towne, ATCC VR-977) sont ajoutés à 2 ml de tampon de lyse (NucliSENS lysis buffer BMX 200292, bioMérieux, Marcy l'Etoile, France)

2. 10μL de solution de contrôle d'extraction et d'inhibition (IC2, contenu dans la trousse de quantification CMV R-gene® (bioMérieux, Marcy l'Etoile, France)) sont ajoutés dans le tube de tampon de lyse contenant l'échantillon de sang.

2' En parallèle dans d'autres tubes, des gammes témoins de $10^{e3}$ à $10^{e6}$ copies du CMV sont réalisées.

3. L'échantillon est soumis à un mélange par vortex pendant quelques secondes puis laissé au repos à température ambiante pendant 10 minutes.

4. On ajoute 50 μl de particules magnétiques à 50 mg/ml dans de l'eau à la solution préparée dans l'exemple 3, soit 2.5 mg et on agite par vortex immédiatement pendant 5 secondes.

5. La suspension est incubée au repos pendant 10 min sans agitation avant d'être transférée dans une navette easyMAG® où les particules magnétiques vont être lavées avant d'en éluer les acides nucléiques.

a. Brièvement, les particules sont lavées dans l'automate avec quelques ml de tampon NucliSENS easyMAG Extraction Buffer 1 (Ref. BMX 280130, bioMérieux, Marcy l'étoile, France))
b. Puis reprises avec quelques ml d'une solution d'éthanol à 50 %,
c. L'élution se fait finalement avec 50 μl de tampon NucliSENS easyMAG Extraction Buffer 3 (ref BMX 280132, bioMérieux, Marcy l'étoile, France) à 70°C pendant 5 minutes

6- L'ADN de CMV extrait est ensuite amplifié et quantifié en utilisant le protocole et les réactifs de la trousse de quantification CMV R-gene® sur un thermocycler BIO-RAD CFX-96 (BioRAD , Hercules, Californie).

<u>Conclusion</u> : On démontre, sur la figure 16, que l'ADN du virus CMV (courbes avec cercle) est amplifié avec une meilleure sensibilité dans le cas de l'utilisation du complexe tri-couches selon l'invention (la particule doublement enrobée AL1013 (magnétite /Ludox 6 % poids/poids / PPi 6,8%)) par rapport à l'utilisation de la particule simplement enrobée AL 1001 magnétite/Ludox 6 % poids/poids) avec respectivement un Cq avancé à 33.7 contre un Cq de 35.5 dans le cas de l'amplification des acides nucléiques extraits avec la particules de magnétite recouverte uniquement de Ludox® 6%. Il en va exactement de même pour l'amplification des acides nucléiques retenus par un autre complexe selon l'invention (les particules doublement enrobées : AL1015 (magnétite/bentonite 30 % / PPi 6,8 %)). On observe également une amplification détectée avec bien plus de sensibilité (Cq = 34.8) alors qu'en utilisant un complexe bi-couches seulement, à savoir les particules AL 1014 (AL1014 magnétite /Bentonite 30%) on n'observe pas d'amplification.

Sur chaque graphe, on observe 4 courbes sans cercle correspondant aux courbes observées avec l'amplification des gammes témoins qui contiennent de $10^{e3}$ à $10^{e6}$ copies du CMV.

On montre, une nouvelle fois, l'effet hautement avantageux, voire synergique du double enrobage sur le composé magnétique pour former les complexe tri-couches selon l'invention.

A noter que la magnétite seule dans ces conditions d'utilisation n'est à l'origine d'aucune amplification / détection du virus CMV

**Références bibliographiques**

- Rapid and simple method for purification of nucleic acids. », Boom, Journal of Clinical Microbiology, 1990 p495
- Magnetic particles for the separation and purification of Nucleic acids" S. Berensmeier, Applied Microbial Biotechnology 2006 73 495-504
- The use of magnetic nanoparticles in the development of new molecular detection systems, I. J. Bruce, Journal of Nanosciences and nanotechnology
- Optimization of influencing factors of nucleic acid adsorption onto silica-coated magnetic particles: Application to viral nucleic acid extraction from serum, Ning Sun and al., Journal of Chromatography A, 1325 (2014) 31-39
- Stability constants of metal-ion complexes, Lars Gunnar Sillén and Arthur Earl Martell, Edition 1971, Chemical Society,
- G Pourroy, Chem. Comm. 2010 46 985-987
- G Pourroy, Chem. Mater., 2008, 20 (18), pp 5869-5875
- Isolation of genomic DNA using magnetic cobalt ferrite and silica particles, B.Rittch, J. of Chromatography A, 2004, 43-48
- Ferrite supports for the isolation of DNA from complex samples and polymerase chain reaction amplification, D. Horak, J, of Chromatography A, 2005, 93-98
- T. Sugimoto and E. Matjevic, Journal of Colloids and Interface Science, 1980, 74, P227-243
- R. Massart, IEEE Trans. Magn. 1981, 17, p1247-1248
- Maity, Journal of Magnetic Materials 321 1256 (2009)
- The Influence of Particle Size, Shape and Particle Size Distribution on Properties of Magnetites for the Production of Toners, Journal of Imaging Science and Technology, November/December 2000, vol. 44, no. 6; p. 508-513
- W. Stöber, Journal of Colloids and Interface Science 1968, 26, p62-69

**Revendications**

1. Complexe tri-couches comprenant :

   - une première couche comprenant au moins un composé magnétique,
   - une deuxième couche recouvrant partiellement la première couche et comprenant au moins un composé inorganique silicaté,
   - une troisième couche recouvrant, au moins partiellement, la deuxième couche et comprenant au moins un composé ayant une affinité pour ledit au moins un composé magnétique et/ou pour ledit au moins un composé inorganique silicaté choisi parmi l'acide citrique et ses sels, les ions phosphates, pyrophosphates, triphosphates, polyphosphates, les acides phosphoniques, les phosphonates, les phosphonates ou les acides phosphoniques couplés à des molécules organiques, les composés de la famille des acides phosphoriques, des sulfonates, les composés de la famille des détergents et/ou les composés de la famille des acides carboxyliques.

2. Complexe tri-couches selon revendication 1, **caractérisé en ce que** ledit au moins un composé magnétique est choisi parmi les métaux et les oxydes métalliques.

3. Complexe tri-couches selon la revendication 1 ou 2, **caractérisé en ce que** ledit composé magnétique est choisi parmi la magnétite, la maghémite et les ferrites.

4. Complexe tri-couches selon l'une quelconque des revendications 1 à 3, **caractérisé en ce que** ledit au moins un composé inorganique silicaté est choisi parmi les silicates, les silicates de magnésium ou de sodium ou de potassium ou de lithium ou de calcium, le talc, les alumino-silicates, le kaolin, la bentonite, les nanoparticules de silice entre 0,1 et 20 nm, de préférence entre 1 nm et 20 nm, les nanoparticules de silice mésoporeuses, les nanoparticules magnétiques recouvertes de silice, les nanoparticules de silice citées précédemment dont la silice est liée à des groupements organiques ou inorganiques.

5. Complexe tri-couches selon la revendication 4, **caractérisée en ce que** ledit au moins un composé inorganique silicaté est constitué de bentonite ou de nanoparticules de silice entre 0,1 nm et 20 nm, de préférence entre 1 et 20 nm.

6. Complexe tri-couches selon l'une quelconque des revendications précédentes, **caractérisé en ce que** ledit au moins un composé inorganique silicaté est sous forme particulaire tel qu'un grain, un feuillet, une fibre, une aiguille, une nano-particule..., de préférence sous forme nano-particulaire, ou sous forme non-soluble dans un solvant aqueux, un mélange solvant aqueux-solvant organique ou un solvant organique.

7. Complexe tri-couches selon la revendication 6, **caractérisé en ce que** les particules ou formes non solubles du composé inorganique silicaté ont une taille comprise entre 0,1 et 20 nm, de préférence entre 1 nm et 20 nm, préférentiellement entre 2 et 10 nm, encore plus préférentiellement entre 6 et 8 nm.

8. Complexe tri-couches selon l'une quelconque des revendications précédentes, **caractérisé en ce que** ledit au moins un composé ayant une affinité pour ledit au moins un composé magnétique et/ou pour ledit au moins un composé inorganique silicaté est de nature organique.

9. Complexe tri-couches selon l'une quelconque des revendications précédentes, **caractérisé en ce que** ledit au moins un composé ayant une affinité pour ledit au moins un composé magnétique et/ou pour ledit au moins un composé inorganique silicaté est de nature inorganique.

10. Complexe tri-couches selon l'une des revendications précédentes, **caractérisé en ce que** ledit au moins un composé ayant une affinité pour ledit au moins un composé magnétique et/ou pour ledit au moins un composé inorganique silicaté est constitué de monophosphates, de pyrophosphates et/ou de triphosphates.

11. Complexe tri-couches selon l'une quelconque des revendications précédentes, **caractérisé en ce qu'**il comprend en outre un support solide sous toute ou partie de la première couche.

12. Complexe selon la revendication 11, **caractérisé en ce que** le support est un support plan, un support creux, une galette, une aiguille, une membrane, une plaque, un cône, un tube, une bille, une particule, de préférence une particule.

13. Complexe tri-couches selon l'une quelconque des revendications précédentes, **caractérisé en ce que** le complexe est sous forme de particule et **en ce que** ladite troisième couche se situe à l'extérieur de la particule.

14. Complexe selon la revendication 13, **caractérisé en ce que** la première couche constitue le cœur de la particule et a une taille comprise entre 2 et 400 nm, de préférence entre 50 et 100 nm.

15. Méthode de préparation d'au moins un complexe tri-couches tel que défini dans l'une quelconque des revendications précédentes, **caractérisée en ce qu'**elle comprend au moins les étapes suivantes :

a0) éventuellement mettre en contact un support tel que défini dans la revendication 12 avec au moins un composé magnétique tel que défini dans la revendication 2 ou 3 de sorte que le au moins un composé magnétique se fixe ou se lie au support,
a) mettre en contact le résultat de l'étape a0) ou au moins un composé magnétique tel que défini dans la revendication 2 ou 3 avec au moins un composé inorganique silicaté tel que défini dans l'une quelconque des revendications 4 à 7, de sorte qu'une interaction électrostatique et/ou une liaison covalente et/ou une liaison de coordination se fasse entre ledit au moins un composé magnétique et ledit au moins un composé inorganique silicaté et de sorte que la couche dudit au moins un composé inorganique silicaté recouvre partiellement la couche dudit au moins un composé magnétique,

b) mettre en contact le résultat de l'étape a) avec au moins un composé ayant une affinité pour ledit au moins un composé magnétique et/ou pour ledit au moins un composé inorganique silicaté tel que défini dans l'une quelconque des revendications 8 à 10, de préférence en milieu aqueux, de sorte que ledit au moins un composé ayant une affinité pour ledit au moins un composé magnétique et/ou pour ledit au moins un composé inorganique silicaté adhère et se positionne au-dessus de la couche dudit au moins un composé inorganique silicaté du complexe bi-couches, avec ou sans support.

16. Méthode de préparation d'un complexe selon l'une quelconque des revendications 1 à 15 comprenant au moins les étapes suivantes :

a) mettre en contact au moins un composé magnétique tel que défini dans la revendication 2 ou 3 avec au moins un composé inorganique silicaté tel que défini dans l'une quelconque des revendications 4 à 7, ledit composé inorganique silicaté se trouvant déjà sous forme particulaire, nano-particulaire ou sous forme non-soluble, de sorte qu'une interaction électrostatique et/ou une liaison covalente et/ou une liaison de coordination se fasse entre lesdits au moins deux composés et que les particules ou formes non-solubles recouvrent partiellement la particule d'au moins un composé magnétique constituant le cœur,

b) mettre en contact la particule enrobée obtenue à l'issue de l'étape a) avec au moins un composé ayant une affinité pour ledit au moins un composé magnétique et/ou pour ledit au moins un composé inorganique silicaté tel que défini dans l'une quelconque des revendications 8 à 10, de préférence en milieu aqueux, de sorte qu'une interaction électrostatique et/ou une liaison covalente et/ou une liaison de coordination se fasse entre ledit au moins un composé ayant une affinité pour ledit au moins un composé inorganique silicaté et/ou ledit au moins un composé magnétique et ledit au moins un composé inorganique silicaté du complexe bi-couches obtenu à l'issue de l'étape a) ou ledit au moins un composé magnétique du complexe bi-couches obtenu à l'issue de l'étape a) et que ledit au moins un composé ayant une affinité pour ledit au moins un composé inorganique silicaté et/ou ledit au moins composé magnétique adhère à la surface dudit au moins un composé inorganique silicaté ou au cœur de la particule finale.

17. Méthode de purification de micro-organismes et/ou biomolécules ou d'extraction biomolécules, de préférence d'acides nucléiques, à partir d'un échantillon, dans laquelle on met en œuvre au moins un complexe tel que défini dans l'une quelconque des revendications 1 à 14 ou tel qu'obtenu selon l'une des revendications 15 ou 16.

18. Méthode de détection et/ou de quantification d'acides nucléiques cibles, à partir d'un échantillon susceptible de contenir lesdits acides nucléiques cibles, comprenant les étapes suivantes :

1. l'extraction des acides nucléiques d'un échantillon par la mise en œuvre de la méthode telle que définie dans la revendication 17,
2. la détection et/ou la quantification des acides nucléiques cibles par des techniques de détection et/ou de quantification classiques.

19. Méthode de détection et/ou de quantification d'acides nucléiques cibles selon la revendication 18, **caractérisée en ce qu'**entre l'étape d'extraction et l'étape de détection des acides nucléiques, il y ait une étape d'élution des acides nucléiques du complexe mis en œuvre dans l'étape 1) et/ou une étape d'amplification des acides nucléiques par des techniques classiques.

20. Méthode de lyse de micro-organismes et/ou de cellules et/ou de tissus, à partir d'un échantillon, **caractérisée en ce qu'**elle consiste à mettre en contact au moins un échantillon avec au moins un complexe tel que défini dans l'une quelconque des revendications 1 à 14 et dans lequel ledit au moins un composé inorganique silicaté et/ou ledit au moins un composé ayant une affinité pour ledit au moins un composé magnétique et/ou pour ledit au moins un composé inorganique silicaté comprend au moins un agent de la famille des détergents permettant la lyse.

21. Méthode selon la revendication 20, dans laquelle ledit au moins un composé inorganique silicaté comprend des nanoparticules de silice entre 0,1 nm et 20 nm, de préférence entre 1nm et 20nm, dont la silice est liée à au moins une saponine et/ou le composé ayant une affinité pour ledit au moins un composé magnétique et/ou pour ledit au moins un composé inorganique silicaté est choisi parmi la dopamine et ses dérivés, les catechols et leurs dérivés, les acides phosphoniques, les phosphonates, les phosphates et les composés de la famille des acides carboxyliques (de préférence l'acide citrique et ses sels) couplés à au moins une saponine.

22. Trousse de diagnostic moléculaire comprenant au moins un complexe tel que défini dans l'une quelconque des revendications 1 à 14.

23. Trousse de diagnostic moléculaire selon la revendication 22 comprenant en outre :

- des réactifs permettant l'amplification spécifique des acides nucléiques susceptibles ou suspectés d'être contenus dans l'échantillon à tester, et/ou
- des réactifs permettant la détection et/ou la quantification des acides nucléiques susceptibles ou suspectés d'être contenus dans l'échantillon à tester.

**Patentansprüche**

1. Dreischichtiger Komplex, umfassend:

- eine erste Schicht, die mindestens eine magnetische Verbindung umfasst,
- eine zweite Schicht, die die erste Schicht teilweise bedeckt und mindestens eine anorganische Silikatverbindung umfasst,
- eine dritte Schicht, die die zweite Schicht zumindest teilweise bedeckt und mindestens eine Verbindung umfasst, die eine Affinität für die mindestens eine magnetische Verbindung und/oder für die mindestens eine anorganische Silikatverbindung besitzt, ausgewählt aus Zitronensäure und ihren Salzen, Phosphat-, Pyrophosphat-, Triphosphat-, Polyphosphationen, Phosphonsäuren, Phosphonaten, an organische Moleküle gekoppelten Phosphonaten oder Phosphonsäuren, Verbindungen aus der Familie der Phosphorsäuren, der Sulfonate, Verbindungen aus der Familie der Detergentien und/oder Verbindungen aus der Familie der Carbonsäuren.

2. Dreischichtiger Komplex nach Anspruch 1, **dadurch gekennzeichnet, dass** die mindestens eine magnetische Verbindung aus Metallen und Metalloxiden ausgewählt ist.

3. Dreischichtiger Komplex nach Anspruch 1 oder 2, **dadurch gekennzeichnet, dass** die magnetische Verbindung aus Magnetit, Maghemit und Ferriten ausgewählt ist.

4. Dreischichtiger Komplex nach einem der Ansprüche 1 bis 3, **dadurch gekennzeichnet, dass** die mindestens eine anorganische Silikatverbindung aus Silikaten, Magnesium- oder Natrium- oder Kalium- oder Lithium- oder Calciumsilikaten, Talk, Alumosilikaten, Kaolin, Bentonit, Siliziumdioxid-Nanopartikeln zwischen 0,1 und 20 nm, vorzugsweise zwischen 1 nm und 20 nm, mesoporösen Siliziumdioxid-Nanopartikeln, mit Siliziumdioxid bedeckten magnetischen Nanopartikeln, den vorstehend genannten Siliziumdioxid-Nanopartikeln, in denen das Siliziumdioxid an organische oder anorganische Gruppierungen gebunden ist, ausgewählt ist.

5. Dreischichtiger Komplex nach Anspruch 4, **dadurch gekennzeichnet, dass** die mindestens eine anorganische Silikatverbindung aus Bentonit oder Siliziumdioxid-Nanopartikeln zwischen 0,1 nm und 20 nm, vorzugsweise zwischen 1 nm und 20 nm, besteht.

6. Dreischichtiger Komplex nach einem der vorhergehenden Ansprüche, **dadurch gekennzeichnet, dass** die mindestens eine anorganische Silikatverbindung in partikulärer Form, wie ein Korn, ein Blättchen, eine Faser, eine Nadel, ein Nanopartikel..., vorzugsweise in nanopartikulärer Form, oder in einer in einem wässrigen Lösungsmittel, einem Gemisch von wässrigem Lösungsmittel/organischem Lösungsmittel oder einem organischen Lösungsmittel unlöslichen Form vorliegt.

7. Dreischichtiger Komplex nach Anspruch 6, **dadurch gekennzeichnet, dass** die Partikel oder unlöslichen Formen der anorganischen Silikatverbindung eine Größe zwischen 0,1 und 20 nm, vorzugsweise zwischen 1 nm und 20 nm, bevorzugt zwischen 2 und 10 nm, noch stärker bevorzugt zwischen 6 und 8 nm aufweisen.

8. Dreischichtiger Komplex nach einem der vorhergehenden Ansprüche, **dadurch gekennzeichnet, dass** die mindestens eine Verbindung mit einer Affinität für die mindestens eine magnetische Verbindung und/oder für die mindestens eine anorganische Silikatverbindung organischer Natur ist.

9. Dreischichtiger Komplex nach einem der vorhergehenden Ansprüche, **dadurch gekennzeichnet, dass** die mindestens eine Verbindung mit einer Affinität für die mindestens eine magnetische Verbindung und/oder für die mindestens eine anorganische Silikatverbindung anorganischer Natur ist.

10. Dreischichtiger Komplex nach einem der vorhergehenden Ansprüche, **dadurch gekennzeichnet, dass** die mindestens eine Verbindung mit einer Affinität für die mindestens eine magnetische Verbindung und/oder für die mindestens eine anorganische Silikatverbindung aus Monophosphaten, Pyrophosphaten und/oder Triphosphaten besteht.

11. Dreischichtiger Komplex nach einem der vorhergehenden Ansprüche, **dadurch gekennzeichnet, dass** er außerdem einen festen Träger unter der gesamten oder einem Teil der ersten Schicht umfasst.

12. Dreischichtiger Komplex nach Anspruch 11, **dadurch gekennzeichnet, dass** der Träger ein ebener Träger, ein Hohlträger, ein Wafer, eine Nadel, eine Membran, eine Platte, ein Kegel, eine Röhre, ein Kügelchen, ein Partikel, vorzugsweise ein Partikel, ist.

13. Dreischichtiger Komplex nach einem der vorhergehenden Ansprüche, **dadurch gekennzeichnet, dass** der Komplex in Partikelform vorliegt, und dadurch, dass sich die dritte Schicht auf der Außenseite des Partikels befindet.

14. Komplex nach Anspruch 13, **dadurch gekennzeichnet, dass** die erste Schicht den Kern des Partikels bildet und eine Größe zwischen 2 und 400 nm, vorzugsweise zwischen 50 und 100 nm aufweist.

15. Verfahren zur Herstellung mindestens eines dreischichtigen Komplexes nach einem der vorhergehenden Ansprüche, **dadurch gekennzeichnet, dass** es mindestens die folgenden Schritte umfasst:

a0) gegebenenfalls Inkontaktbringen eines Trägers nach Anspruch 12 mit mindestens einer magnetischen Verbindung nach Anspruch 2 oder 3, so dass sich die mindestens eine magnetische Verbindung am Träger festsetzt oder daran bindet,
a) Inkontaktbringen des Ergebnisses von Schritt a0) oder mindestens einer magnetischen Verbindung nach Anspruch 2 oder 3 mit mindestens einer anorganischen Silikatverbindung nach einem der Ansprüche 4 bis 7, so dass eine elektrostatische Wechselwirkung und/oder eine kovalente Bindung und/oder eine koordinative Bindung zwischen der mindestens einen magnetischen Verbindung und der mindestens einen anorganischen Silikatverbindung auftritt und so dass die Schicht der mindestens einen anorganischen Silikatverbindung die Schicht der mindestens einen magnetischen Verbindung teilweise bedeckt, und
b) Inkontaktbringen des Ergebnisses von Schritt a) mit mindestens einer Verbindung mit einer Affinität für die mindestens eine magnetische Verbindung und/oder für die mindestens eine anorganische Silikatverbindung nach einem der Ansprüche 8 bis 10, vorzugsweise in wässrigem Medium, so dass die mindestens eine Verbindung mit einer Affinität für die mindestens eine magnetische Verbindung und/oder für die mindestens eine anorganische Silikatverbindung über der Schicht der mindestens einen anorganischen Silikatverbindung des zweischichtigen Komplexes, mit oder ohne Träger, haftet und sich darüber positioniert.

16. Verfahren zur Herstellung eines Komplexes nach einem der Ansprüche 1 bis 15, das mindestens die folgenden Schritte umfasst:

a) Inkontaktbringen mindestens einer magnetischen Verbindung nach Anspruch 2 oder 3 mit mindestens einer anorganischen Silikatverbindung nach einem der Ansprüche 4 bis 7, wobei die anorganische Silikatverbindung bereits in partikulärer, nanopartikulärer oder unlöslicher Form vorliegt, so dass eine elektrostatische Wechselwirkung und/oder eine kovalente Bindung und/oder eine koordinative Bindung zwischen den mindestens zwei Verbindungen auftritt und so dass die Partikel oder unlöslichen Formen das den Kern bildende Partikel der mindestens einer magnetischen Verbindung teilweise bedecken, und
b) Inkontaktbringen des nach Schritt a) erhaltenen umhüllten Partikels mit mindestens einer Verbindung mit einer Affinität für die mindestens eine magnetische Verbindung und/oder für die mindestens eine anorganische Silikatverbindung nach einem der Ansprüche 8 bis 10, vorzugsweise in wässrigem Medium, so dass eine elektrostatische Wechselwirkung und/oder eine kovalente Bindung und/oder eine koordinative Bindung zwischen der mindestens einen Verbindung mit einer Affinität für die mindestens eine anorganische Silikatverbindung und/oder die mindestens eine magnetische Verbindung und der mindestens einen anorganischen Silikatverbindung des nach Schritt a) erhaltenen zweischichtigen Komplexes oder der mindestens einen magnetischen Verbindung des nach Schritt a) erhaltenen zweischichtigen Komplexes auftritt und so dass die mindestens eine

31

Verbindung mit einer Affinität für die mindestens eine anorganische Silikatverbindung und/oder die mindestens eine magnetische Verbindung an der Oberfläche der mindestens einen anorganischen Silikatverbindung und/oder am Kern des endgültigen Partikels haftet.

17. Verfahren zum Reinigen von Mikroorganismen und/oder Biomolekülen oder zur Extraktion von Biomolekülen, vorzugsweise Nukleinsäuren, aus einer Probe, bei dem man mindestens einen Komplex nach einem der Ansprüche 1 bis 14 oder erhalten nach einem der Ansprüche 15 oder 16 einsetzt.

18. Verfahren zum Nachweis und/oder zur Quantifizierung von Zielnukleinsäuren aus einer Probe, die die Zielnukleinsäuren enthalten kann, das die folgenden Schritte umfasst:

1. Extraktion der Nukleinsäuren aus einer Probe durch Einsatz des Verfahrens nach Anspruch 17,
2. Nachweis und/oder zur Quantifizierung der Zielnukleinsäuren durch herkömmliche Nachweis- und/oder Quantifizierungstechniken.

19. Verfahren zum Nachweis und/oder zur Quantifizierung von Zielnukleinsäuren nach Anspruch 18, **dadurch gekennzeichnet, dass** zwischen dem Schritt der Extraktion und dem Schritt des Nachweisens der Nukleinsäuren ein Schritt der Elution der Nukleinsäuren aus dem in Schritt 1) eingesetzten Komplex und/oder ein Schritt der Amplifikation der Nukleinsäuren durch herkömmliche Techniken erfolgt.

20. Verfahren zur Lyse von Mikroorganismen und/oder Zellen und/oder Geweben aus einer Probe, **dadurch gekennzeichnet, dass** es aus dem Inkontaktbringen mindestens eine Probe mit mindestens einem Komplex nach einem der Ansprüche 1 bis 14 besteht und wobei die mindestens eine anorganische Silikatverbindung und/oder die mindestens eine Verbindung mit einer Affinität für die mindestens eine magnetische Verbindung und/oder für die mindestens eine anorganische Silikatverbindung mindestens ein Mittel aus der Familie der Detergentien umfasst, das die Lyse ermöglicht.

21. Verfahren nach Anspruch 20, wobei die mindestens eine anorganische Silikatverbindung Siliziumdioxid-Nanopartikel zwischen 0,1 nm und 20 nm, vorzugsweise zwischen 1 nm und 20 nm umfasst, worin das Siliziumdioxid an mindestens ein Saponin gebunden ist, und/oder die Verbindung mit einer Affinität für die mindestens eine magnetische Verbindung und/oder für die mindestens eine anorganische Silikatverbindung aus Dopamin und seinen Derivaten, Brenzkatechinen und ihren Derivaten, Phosphonsäuren, Phosphonaten, Phosphaten und Verbindungen aus der Familie der Carbonsäuren (vorzugsweise Zitronensäure und ihren Salzen), die an mindestens ein Saponin gekoppelt sind, ausgewählt ist.

22. Molekulardiagnostik-Kit, das mindestens einen Komplex nach einem der Ansprüche 1 bis 14 umfasst.

23. Molekulardiagnostik-Kit nach Anspruch 22, außerdem umfassend:

- Reagenzien, die die spezifische Amplifikation von Nukleinsäuren ermöglichen, die in der zu testenden Probe enthalten sein können oder vermutlich enthalten sind, und/oder
- Reagenzien, die den Nachweis und/oder die Quantifizierung der Nukleinsäuren ermöglichen, die in der zu testenden Probe enthalten sein können oder vermutlich enthalten sind.

**Claims**

1. Three-layer complex comprising:

- a first layer comprising at least one magnetic compound,
- a second layer partially covering the first layer and comprising at least one inorganic silicate compound,
- a third layer at least partially covering the second layer and comprising at least one compound having an affinity for said at least one magnetic compound and/or for said at least one inorganic silicate compound, chosen from citric acid and salts thereof, phosphate, pyrophosphate, triphosphate or polyphosphate ions, phosphonic acids, phosphonates, phosphonates or phosphonic acids coupled to organic molecules, compounds of the phosphoric acid family, sulfonates, compounds of the detergent family and/or compounds of the carboxylic acid family.

**2.** Three-layer complex according to Claim 1, **characterized in that** said at least one magnetic compound is chosen from metals and metal oxides.

**3.** Three-layer complex according to Claim 1 or 2, **characterized in that** said magnetic compound is chosen from magnetite, maghemite and ferrites.

**4.** Three-layer complex according to any one of Claims 1 to 3, **characterized in that** said at least one inorganic silicate compound is chosen from silicates, magnesium, sodium, potassium, lithium or calcium silicates, talc, aluminosilicates, kaolin, bentonite, silica nanoparticles between 0.1 and 20 nm, preferably between 1 nm and 20 nm, mesoporous silica nanoparticles, magnetic nanoparticles covered with silica, and the silica nanoparticles previously mentioned, the silica of which is bonded to organic or inorganic groups.

**5.** Three-layer complex according to Claim 4, **characterized in that** said at least one inorganic silicate compound consists of bentonite or of silica nanoparticles between 0.1 nm and 20 nm, preferably between 1 and 20 nm.

**6.** Three-layer complex according to any one of the preceding claims, **characterized in that** said at least one inorganic silicate compound is in particulate form such as a grain, a sheet, a fibre, a needle, a nanoparticle, etc., preferably in nanoparticulate form, or in a form which is insoluble in an aqueous solvent, an aqueous solvent/organic solvent mixture or an organic solvent.

**7.** Three-layer complex according to Claim 6, **characterized in that** the particles or insoluble forms of the inorganic silicate compound have a size of between 0.1 and 20 nm, preferably between 1 nm and 20 nm, preferentially between 2 and 10 nm, even more preferentially between 6 and 8 nm.

**8.** Three-layer complex according to any one of the preceding claims, **characterized in that** said at least one compound having an affinity for said at least one magnetic compound and/or for said at least one inorganic silicate compound is organic in nature.

**9.** Three-layer complex according to any one of the preceding claims, **characterized in that** said at least one compound having an affinity for said at least one magnetic compound and/or for said at least one inorganic silicate compound is inorganic in nature.

**10.** Three-layer complex according to one of the preceding claims, **characterized in that** said at least one compound having an affinity for said at least one magnetic compound and/or for said at least one inorganic silicate compound consists of monophosphates, of pyrophosphates and/or of triphosphates.

**11.** Three-layer complex according to any one of the preceding claims, **characterized in that** it also comprises a solid support under all or part of the first layer.

**12.** Complex according to Claim 11, **characterized in that** the support is a flat support, a hollow support, a round piece, a needle, a membrane, a plaque, a cone, a tube, a bead, a particle, preferably a particle.

**13.** Three-layer complex according to any one of the preceding claims, **characterized in that** the complex is in the form of a particle and **in that** said third layer is on the outside of the particle.

**14.** Complex according to Claim 13, **characterized in that** the first layer constitutes the core of the particle and has a size of between 2 and 400 nm, preferably between 50 and 100 nm.

**15.** Method for preparing at least one three-layer complex as defined in any one of the preceding claims, **characterized in that** it comprises at least the following steps:

a0) optionally bringing a support as defined in Claim 12 into contact with at least one magnetic compound as defined in Claim 2 or 3 such that the at least one magnetic compound attaches or bonds to the support,
a) bringing the result of step a0) or at least one magnetic compound as defined in Claim 2 or 3 into contact with at least one inorganic silicate compound as defined in any one of Claims 4 to 7, such that an electrostatic interaction and/or a covalent bond and/or a coordination bond occurs between said at least one magnetic compound and said at least one inorganic silicate compound and such that the layer of said at least one inorganic silicate compound partially covers the layer of said at least one magnetic compound,

b) bringing the result of step a) into contact with at least one compound having an affinity to said at least one magnetic compound and/or for said at least one inorganic silicate compound as defined in any one of Claims 8 to 10, preferably in an aqueous medium, such that said at least one compound having an affinity for said at least one magnetic compound and/or for said at least one inorganic silicate compound adheres and positions itself above the layer of said at least one inorganic silicate compound of the two-layer complex, with or without support.

16. Method for preparing a complex according to any one of Claims 1 to 15, comprising at least the following steps:

a) bringing at least one magnetic compound as defined in Claim 2 or 3 into contact with at least one inorganic silicate compound as defined in any one of Claims 4 to 7, said inorganic silicate compound already being in particulate form, in nanoparticulate form or in insoluble form, such that an electrostatic interaction and/or a covalent bond and/or a coordination bond occurs between said at least two compounds and that the particles or insoluble forms partially cover the particle of at least one magnetic compound constituting the core,

b) bringing the coated particle obtained at the end of step a) into contact with at least one compound having an affinity for said at least one magnetic compound and/or for said at least one inorganic silicate compound as defined in any one of Claims 8 to 10, preferably in an aqueous medium, such that an electrostatic interaction and/or a covalent bond and/or a coordination bond occurs between said at least one compound having an affinity for said at least one inorganic silicate compound and/or said at least one magnetic compound and said at least one inorganic silicate compound of the two-layer complex obtained at the end of step a) or said at least one magnetic compound of the two-layer complex obtained at the end of step a) and that said at least one compound having an affinity for said at least one inorganic silicate compound and/or said at least one magnetic compound adheres to the surface of said at least one inorganic silicate compound or to the core of the final particle.

17. Method for purifying microorganisms and/or biomolecules or for extracting biomolecules, preferably nucleic acids, from a sample, in which at least one complex as defined in any one of Claims 1 to 14 or as obtained as claimed in either of Claims 15 and 16 is used.

18. Method for detecting and/or quantifying target nucleic acids from a sample that may contain said target nucleic acids, comprising the following steps:

1. extracting the nucleic acids from a sample by carrying out the method as defined in Claim 17,
2. detecting and/or quantifying the target nucleic acids by means of conventional detection and/or quantification techniques.

19. Method for detecting and/or quantifying target nucleic acids according to Claim 18, **characterized in that**, between the nucleic acid extraction step and the nucleic acid detection step, there is a step of eluting the nucleic acids from the complex used in step 1) and/or a step of amplifying the nucleic acids by conventional techniques.

20. Method for lysing microorganisms and/or cells and/or tissues, from a sample, **characterized in that** it consists in bringing at least one sample into contact with at least one complex as defined in any one of Claims 1 to 14 and in which said at least one inorganic silicate compound and/or said at least one compound having an affinity for said at least one magnetic compound and/or for said at least one inorganic silicate compound comprises at least one agent of the detergent family enabling lysis.

21. Method according to Claim 20, in which said at least one inorganic silicate compound comprises silica nanoparticles between 0.1 nm and 20 nm, preferably between 1 nm and 20 nm, of which the silica is bonded to at least one saponin and/or the compound having an affinity for said at least one magnetic compound and/or for said at least one inorganic silicate compound is chosen from dopamine and derivatives thereof, catechols and derivatives thereof, phosphonic acids, phosphonates, phosphates and compounds of the carboxylic acid family (preferably citric acid and salts thereof) coupled to at least one saponin.

22. Molecular diagnostic kit comprising at least one complex as defined in any one of Claims 1 to 14.

23. Molecular diagnostic kit according to Claim 22, also comprising:

- reagents which allow the specific amplification of the nucleic acids that may be or that are suspected of being contained in the test sample, and/or

- reagents which allow the detection and/or quantification of the nucleic acids that may be or that are suspected of being contained in the test sample.

Figure 1

Complexe tri-couches

Support solide

Figure 2

1ère adsorption

L

2ème adsorption

Figure 3

100 nm

Figure 4

Figure 5

Figure 6

Figure 7A

Figure 7B

Figure 8

Figure 9

Figure 10

Figure 11

Figure 12

A

B

Figure 13

Figure 14

A

B

Figure 15

A

B

Figure 16

**A5/A8: Silice AL1001 magnétite @Ludox 6 %**

CMV Amplification

**B1/B4: Silice AL1013 magnétite @Ludox 6 % w/w @ PPi 6,8%**

CMV Amplification

| silice | Cq CMV |
|--------|--------|
| 1001 | 35,5 |
| 1013 | 33,7 |
| 1014 | N/A |
| 1015 | 34,8 |
| LS | 33,45 |

**B5/B8: Silice AL1014 magnétite @Bentonite 30%**

CMV Amplification

**C1/C4: Silice AL1015 magnétite @bentonite 30 % @ PPi 6,8 %**

CMV Amplification

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5234809 A
- US 20100009375 A1
- US 20050287583 A1
- US 692403382 B
- US 20110186524 A1
- US 2009182120 A
- EP 1674571 A
- US 6936414 B
- US 20070148651 A
- US 5160725 A
- US 4280918 A
- FR 0854549

**Littérature non-brevet citée dans la description**

- *Boom, Journal of Clinical Microbiology,* 1990, 495
- **S. BERENSMEIER.** Magnetic particles for the separation and purification of Nucleic acids. *Applied Microbial Biotechnology,* 2006, vol. 73, 495-504
- **I. J. BRUCE.** The use of magnetic nanoparticles in the development of new molecular detection systems. *Journal of Nanosciences and nanotechnology*
- **NING SUN.** Optimization of influencing factors of nucleic acid adsorption onto silica-coated magnetic particles: Application to viral nucleic acid extraction from serum. *Journal of Chromatography A,* 2014, vol. 1325, 31-39
- **LARS GUNNAR SILLÉN ; ARTHUR EARL MARTELL.** Stability constants of metal-ioncomplexes. Chemical Society, 1971
- **G. POURROY.** *Chem. Comm.,* 2010, vol. 46, 985-987
- *Chem. Mater.,* 2008, vol. 20 (18), 5869-5875
- **RITTICH BOHUSLAV ; B. RITTCH.** Isolation of genomic DNA using magnetic cobalt ferrite and silica particles. *J. of Chromatography A,* 2004, 43-48
- **DAVID HORACK ; D. HORAK.** Ferrite supports for the isolation of DNA from complex samples and polymerase chain reaction amplification. *J. of Chromatography A,* 2005, 93-98
- **R. MASSART.** IEEE Trans. Magn. 1981, vol. 17, 1247-1248
- **T. SUGIMOTO ; E. MATJEVIC.** *Journal of Colloids and Interface Science,* 1980, vol. 74, 227-243
- **MAITY.** *Journal of Magnetic Materials,* 2009, vol. 321, 1256
- The Influence of Particle Size, Shape and Particle Size Distribution on Properties of Magnetites for the Production of Toners. *The Journal of Imaging Science and Technology,* Novembre 2000, vol. 44 (6), 508-513
- **W. STÖBER.** *Journal of Colloids and Interface Science,* 1968, vol. 26, 62-69
- **TYAGI ; KRAMER.** *Nature Biotech,* 1996, vol. 14, 303-308
- **BOOM.** Rapid and simple method for purification of nucleic acids. *Journal of Clinical Microbiology,* 1990, 495
- **LARS GUNNAR SILLÉN ; ARTHUR EARL MARTELL.** Stability constants of metal-ion complexes. Chemical Society, 1971
- **G POURROY.** *Chem. Comm.,* 2010, vol. 46, 985-987
- **G POURROY.** *Chem. Mater.,* 2008, vol. 20 (18), 5869-5875
- **B.RITTCH.** Isolation of genomic DNA using magnetic cobalt ferrite and silica particles. *J. of Chromatography A,* 2004, 43-48
- **D. HORAK.** Ferrite supports for the isolation of DNA from complex samples and polymerase chain reaction amplification. *J, of Chromatography A,* 2005, 93-98
- **R. MASSART.** *IEEE Trans. Magn.,* 1981, vol. 17, 1247-1248
- The Influence of Particle Size, Shape and Particle Size Distribution on Properties of Magnetites for the Production of Toners. *Journal of Imaging Science and Technology,* Novembre 2000, vol. 44 (6), 508-513